# EUROPÄISCHE PATENTSCHRIFT

(11) **EP 1 351 181 B1**
(45) Veröffentlichungstag und Bekanntmachung des Hinweises auf die Patenterteilung: **29.10.2003**
(21) Anmeldenummer: 02012822.9
(22) Anmeldetag: 10.06.2002
(51) Int. Cl.: G06F 19/00

(54) **Computersystem und Verfahren zur Datenerfassung für die Ermittlung des Verlaufs einer chronischen Erkrankung**
Computer system and method for acquiring data to determine the progression of a chronic disease
Procédé et système informatique pour l'acquisition de données pour déterminer l'évolution d'une maladie chronique

(43) Veröffentlichungstag der Anmeldung: 08.10.2003
(73) Patentinhaber: Medvantis Medical Services GmbH, 65201 Wiesbaden (DE)
(72) Erfinder: Jäger-Glogauer, Birgit, 65193 Wiesbaden (DE); Kaeding, André, 98693 Ilmenau (DE)
(74) Vertreter: Richardt, Markus Albert

(56) Entgegenhaltungen:
- EP-A- 1 174 816

## Beschreibung

Die Erfindung betrifft ein Computersystem und ein Verfahren zur Datenerfassung für die Ermittlung des Verlaufs und der Steuerung der Betreuung einer chronischen Erkrankung, beispielsweise von Diabetes oder Asthma, sowie ein entsprechendes Computerprogramm.

Aus dem Stand der Technik sind verschiedene Vorrichtungen und Verfahren für das sogenannte Gesundheitsmanagement bekannt:

Aus US-A- 6,294,999 ist ein System zur Überwachung der Einhaltung einer verordneten Medikamentierung eines Patienten bekannt. Hierzu ist ein Abgabegerät für Medikamente vorgesehen, welches über eine Kommunikationsschnittstelle verfügt. Über dieses Kommunikationsschnittstelle werden Daten hinsichtlich der Entnahme von Medikamenten an Dritte übertragen, wie z. B. an Gesundheitsdienste, Apotheken oder andere Lieferanten von Gesundheitsprodukten und Dienstleistungen. Die Übertragung der Daten erfolgt dabei über ein Computernetzwerk.

Ferner können Daten auch mit verschiedenen anderen Geräten, z.B. Sportgeräten, ausgetauscht werden, um z. B. die Medikamentierung einer sportlichen Betätigung anzupassen. Ähnliche Systeme zur Überwachung der Einhaltung einer bestimmten Medikamentierung sind auch aus US-A-4,616,316 und US-A-5,408,443 bekannt. Entsprechende Geräte werden z. B. von E-pill (www.epill.com) angeboten, wie z. B. ein Pagersystem, welches Nachrichten an Patienten sendet, um diese an die Einnahme von Medikamenten zu bestimmten Uhrzeiten zu erinnern.

Aus US-A-6,234,964 ist ein medizinisches Wissenssystem bekannt, welches für das computerbasierte Langzeitmanagement von Krankheiten, wie z. B. von Asthma und Diabetes, dient. Mittels dieses Systems wird der Gesundheitszustand des Patienten gefördert, indem dem Patienten Informationen angeboten werden und der Gesundheitszustand des Patienten präventiv überwacht wird. Die Überwachung dient als Grundlage zur Optimierung der Therapie.

Aus US-A-6,206,829 ist ein computerbasiertes Verfahren für die medizinische Diagnose über ein Computernetzwerk bekannt. Dabei wird eine medizinische Ferndiagnose über ein Telefon oder ein Computernetzwerk getroffen, indem Anamnesedaten und weitere Daten in einem wissensgestützten medizinischen Diagnosesystem verarbeitet werden. Entsprechende Ansätze sind auch aus US-A-4,712,562, US-A-4,531,527, US-A-4,838,275 und US-A-5,012,411 bekannt.

Aus US-A- 6,171,237 ist ein Überwachungssystem zur Überwachung des Gesundheitszustands eines Patienten bekannt. Dazu werden verschiedene medizinische Daten eines Patienten von für den Patienten konfigurierten Testeinheiten ermittelt und zu einer zentralen Überwachungsstation übertragen. Ähnliche Überwachungssysteme zeigen auch US-A-4,803,625, US-A-4,838,275, US-A-5,231,001 und US-A-5,012,411.

Aus US-A-6,167,362 ist ein elektronisches Gerät zur Information und Warnung eines chronisch kranken Patienten hinsichtlich der Konsequenzen seines Lebensstils und der Einhaltung bzw. Nichteinhaltung einer bestimmten Medikamentierung bekannt. Hierzu erzeugt das Gerät ein virtuelles "Haustier", welches die Konsequenzen eines bestimmten Verhaltens des Patienten symbolisch wiedergibt.

Aus US-A-6,159,147 ist eine Computerkarte zur Erfassung von echtzeitbiologischen Daten bekannt. Dies erlaubt es einen Personal Computer zu einem medizinischen Diagnosegerät auszubauen. Mittels dieser Computerkarte können unterschiedliche physiologische Daten, wie z. B. Blutdruck, Körpertemperatur, Atmungs-Rate, EKG-Signale, Blutalkohol und andere Werte erfasst werden.

Aus US-A- 6,112,182 ist ein Verfahren für das integrierte Management von pharmazeutischen und Gesundheitsdienstleistungen bekannt. Hierzu wird die Medikamentierungshistorie eines Patienten in einer Datenbank gespeichert. Eine aktuelle Verordnung eines Medikaments wird mit der Medikamentierungshistorie verglichen, um eine Plausibilitätsprüfung hinsichtlich der Korrektheit der Verordnung vorzunehmen. Neben der Medikamentierungshistorie können weitere physiologische Daten und verhaltensbezogene Daten des Patienten mit in die Prüfung einbezogen werden.

Aus US-A- 6,108,635 ist ein integriertes krankheitsbezogenes Informationssystem bekannt. Eine Datenbank mit krankheitsbezogenen Daten wird benutzt, um ein bestimmtes Behandlungsverfahren zur validieren.

Aus US-A-6,014,631 ist ein computerimplementiertes System zur Überprüfung der Medikamentierung eines Patienten bekannt. Mittels eines interaktiven Computerprogramms wird die Medikamentierung eines Patienten analysiert, geprüft und gegebenenfalls angepasst. Ein ähnliches Verfahren ist auch aus US-A-4,839,822 bekannt.

Aus US-A-5,940,802 ist ein digitales Krankheitsmanagementsystem bekannt. Mittels Daten, welche den aktuellen Gesundheitszustand eines Patienten angeben, wird eine Risikoanalyse durchgeführt. Basierend auf dieser Risikoanalyse wird entschieden, ob eine sofortige Intervention erforderlich ist.

Aus US 2001 001 2913 A1 ist ein Krankheitsmanagementsystem mit einer Korrelationsbewertung bekannt, welches auf der Berechnung einer Metrik beruht. Basierend auf der Metrik kann eine Therapie des Patienten angepasst werden.

Femer ist von der Firma Nutrion AG, 80798 München, ein tragbares Gerät erhältlich, welches zur Vereinfachung der täglichen Diabetes-Dokumentation dient. Das Gerät ist mit einem elektronischen Tagebuch ausgestattet und erlaubt ferner die Betrachtung und die Analyse der Diabetes-Werte in Diagrammen sowie die Bestimmung des aktuellen Insulinbedarfs auf der Grundlage persönlicher Daten.

Ein gemeinsamer Nachteil der aus dem Stand der Technik vorbekannten Vorrichtungen und Verfahren für das Gesundheitsmanagement ist deren Benutzerunfreundlichkeit.

Der Erfindung liegt daher die Aufgabe zu Grunde, ein verbessertes Verfahren, ein verbessertes Computersystem sowie ein entsprechendes Computerprogrammprodukt zu schaffen. Die der Erfindung zu Grunde liegende Aufgabe wird mit den Merkmalen der unabhängigen Patentansprüche jeweils gelöst. Bevorzugte Ausführungsformen der Erfindung sind in den abhängigen Patentansprüchen angegeben.

Die Erfindung ermöglicht eine bequeme und nutzerfreundliche Datenerfassung für die Ermittlung des Verlaufs und die Steuerung der Betreuung einer chronischen Erkrankung, wie z. B. Asthma oder Diabetes. Hierzu erfolgt zunächst die Eingabe von Anamnesedaten durch den Patienten oder den Arzt, z. B. über eine Internetverbindung in die Datenbank eines Server-Computers.

Nach einer bevorzugten Ausführungsform der Erfindung wird dann durch den Server-Computer eine Auswahl eines typischen Betreuungsablauf-Profils getroffen. Dazu werden die zuvor erhobenen Daten mittels einer Verarbeitungseinheit analysiert, die unter anderem das notwendige medizinische Fachwissen in Form von Regeln und Algorithmen enthält. Die Verarbeitungseinheit kann durch ein Expertensystem, ein neuronales Netz oder ein anderes auf Regeln und/oder Algorithmen basierendes System realisiert werden. Ebenso kann mit Suchbegriffen und boolschen Operatoren gearbeitet werden. Mit Hilfe der Verarbeitungseinheit wird also basierend auf den erhobenen Daten ein möglichst gut passendes typisches Betreuungsablauf-Profil gewählt.

In einer Datenbank sind die typischen Betreuungsablauf-Profile gespeichert. Jedes der typischen Betreuungsablauf-Profile ist dabei einer bestimmten Erkrankung und / oder einer bestimmten Therapie der Erkrankung zugeordnet. Das Wissen um diese Zuordnung wird von den jeweiligen medizinischen Spezialisten erfasst und in formalisierter Form der oben genannten Verarbeitungseinheit zur Verfügung gestellt. Der Grundgedanke ist hierbei, dass die Wahl einer bestimmten Therapieform zur Behandlung einer chronischen Erkrankung die Einhaltung eines typischen Betreuungsablauf-Profils voraussetzt. Von einem solchen typischen Betreuungsablauf-Profil kann jedoch auch individuell abgewichen werden.

Um dem Patienten die Eingabe zu erleichtern, wird zunächst eine Eingabemaske mit dem automatisch ausgewählten typischen Betreuungsablauf-Profil, z. B. auf dem Client-Computer des Patienten, angezeigt. Dieses typische Betreuungsablauf-Profil beinhaltet Eingabefelder, in die bereits Standardwerte eingetragen sind. Dieses Standardwerte bzw. Standardeintragungen können von dem Patienten editiert werden. Ferner können auch weitere Daten zur Modifikation und / oder Ergänzung des typischen Betreuungsablauf-Profils von dem Patienten eingegeben werden. Auf diese Art und Weise entsteht ein individuelles Betreuungsablauf-Profil des betreffenden Patienten. Durch die Eingabe einer Bestätigung wird dieses individuelle Betreuungsablauf-Profil für den betreffenden Patienten auf einer Datenbank des Host-Computers gespeichert.

Im Weiteren kann der Patient dann auf besonders bequeme Art und Weise seine Daten betreffend seinen Krankheitsverlauf eingeben. Hierzu nimmt der Patient, z. B. über den Internet-Browser seines Client-Computers mit dem Server-Computer Kontakt auf und authentifiziert sich, beispielsweise mittels seiner Benutzer-ID und seines Passworts. Weitere Möglichkeiten der Kontaktaufnahme sind die Nutzung lokaler Computerprogramme und Mobiler Computer, die über die entsprechenden Schnittstellen verfügen.

Beispielsweise mit der Benutzer-ID als Schlüssel wird dann serverseitig auf das individuelle Betreuungsablauf-Profil des betreffenden Patienten zugegriffen und dieses in der Form einer Eingabemaske auf dem Client-Computer des Patienten zur Anzeige gebracht. Diese Anzeigemaske beinhaltet bereits die individuellen Standardeintragungen gemäß des individuellen Betreuungsablauf-Profils, welche wiederum editierbar sind, um das individuelle Betreuungsablauf-Profil mit dem tatsächlichen Betreuungsablauf-Profil abzugleichen.

Wenn sich der Patient exakt an dessen definiertes individuelles Betreuungsablauf-Profil gehalten hat, brauchen die individuellen Standardeintragungen in der Eingabemaske lediglich von dem Patienten bestätigt zu werden. Im Falle von Abweichungen ist es nur erforderlich, die entsprechenden Standardeintragungen zu editieren. Dies erspart dem Patienten viel Mühe bei der Dateneingabe, was insbesondere insofern wichtig ist, als diese Datenerfassung regelmäßig und fortlaufend erfolgt.

Neben den verhaltensbezogenen Daten des tatsächlichen Betreuungsablauf-Profils wird ferner zumindest ein aktueller charakteristischer Messwert hinsichtlich der chronischen Erkrankung ermittelt. Dies kann so erfolgen, dass der Patient mittels eines entsprechenden Messgeräts einen charakteristischen physiologischen Messwert ermittelt und diesen dann manuell in die Eingabemaske eingibt. Alternativ kann des betreffende Messgerät auch mit einer Datenschnittstelle ausgerüstet sein, welches eine unmittelbare Übertragung des oder der Messwerte zu dem Client-Computer des Patienten bzw. direkt zu dem Host-Computer ermöglicht.

Die tatsächlichen Betreuungsablauf-Profildaten bestehend aus den verhaltensbezogenen tatsächlichen Betreuungsablauf-Profildaten und dem zumindest einen charakteristischen Messwert werden nach einer bevorzugten Ausführungsform der Erfindung von dem Client-Computer zu dem Host-Computer übertragen und dort in einer Datenbank abgespeichert. Über einen bestimmten Beobachtungszeitraum werden so die tatsächlichen Betreuungsablauf-Profildaten des Patienten gesammelt. Die tatsächlichen Betreuungsablauf-Profildaten können beispielsweise punktuell oder für einen bestimmten Beobachtungszeitraum z. B. für die Zwecke der Risikoanalyse ausgewertet werden. Hierzu kann das regel- und/oder algorithmenbasiertes System unter Nutzung der bereits genannten Verarbeitungseinheit für medizinischen Fachwissen verwendet werden.

Nach einer weiteren bevorzugten Ausführungsform der Erfindung werden die Ergebnisse der Analyse dem Benutzer in symbolischer Form, z. B. mittels einer Farbcodierung, angezeigt. Hierzu kann beispielsweise zwischen drei Bereichen unterschieden werden: Normalbereich, Grenzbereich und Risikobereich. Daten, die in dem Normalbereich liegen, werden beispielsweise grün hinterlegt angezeigt; Daten des Grenzbereichs gelb hinterlegt und Daten des Risikobereichs rot hinterlegt. Wenn Daten im Risikobereich liegen oder anderweitig definierte Trigger erreichen, können weitere automatische Aktionen von dem Computersystem veranlasst werden, wie die Übergabe an das Remindermodul, welches Aktionen wie z. B. das Absenden einer E-Mail-Nachricht und / oder einer SMS-Nachricht an den behandelnden Arzt des Patienten oder einen anderen Dritten, wie z. B. einen Gesundheitsdienstleister, das Versenden von weitergehenden Informationen in Brief- oder Faxform oder andere definierte Reaktionen auslösen kann.

Nach einer weiteren bevorzugten Ausführungsform der Erfindung wird ein tragbares Eingabegerät für die Eingabe des tatsächlichen Betreuungsablauf-Profils verwendet. Nachdem das individuelle Betreuungsablauf-Profil von dem Patienten definiert worden ist, wird dieses z. B. von dem Host-Computer oder von dem Server-Computer auf das tragbare Eingabegerät geladen. Der Benutzer kann dann mittels einer Kalenderfunktion für jeden Tag eine Eingabemaske mit dem individuellen Betreuungsablaufprofil aufrufen. Das individuelle Betreuungsablauf-Profil beinhaltet wiederum Datenfelder, die mit editierbaren Standardeintragungen vorbelegt sind. Der Benutzer kann dann diese Standardeintragungen entweder bestätigen oder zunächst editieren sowie weitere Daten zur Modifikation und / oder Ergänzung des individuellen Betreuungsablauf-Profils eingeben.

Ferner ist in dem tragbaren Eingabegerät vorzugsweise ein Messgerät zur Messung des charakteristischen physiologischen Messwerts integriert, also beispielsweise ein Messgerät zur Bestimmung eines Blutzuckerwertes oder ein sogenanntes Peak-Flowmeter im Falle von Asthma. Dies hat für den Patienten mehrere wesentliche Vorteile:

Die Mobilität des Patienten erhöht sich, da er das tragbare Eingabegerät z. B. auch bei einer Reise mit sich führen kann.

Es ist nur ein einziges Gerät erforderlich, da auch das Messgerät in das Eingabegerät integriert ist.

Die manuelle Eingabe des Messwerts in die Eingabemaske entfällt, da dieser Messwert unmittelbar mittels des Eingabegeräts ermittelt wird.

Nach einer bevorzugten Ausführungsform der Erfindung ist das Eingabegerät an den Client-Computer des Patienten über eine Schnittstelle anschließbar, um eine Synchronisation vorzunehmen. Beispielsweise kann der Patient für einen oder mehrere Tage auf Reisen sein und die täglichen Eingaben seines tatsächlichen Betreuungsablaufprofils mittels des Eingabegeräts vornehmen. Die betreffenden tatsächlichen Betreuungsablauf-Profildaten werden dann in dem Eingabegerät lokal gespeichert. Wenn der Patient von seiner Reise zurückkehrt, schließt er das Eingabegerät an die Schnittstelle zu seinem Client-Computer an, so dass die in dem Eingabegerät lokal gespeicherten tatsächlichen Betreuungsablauf-Profildaten zu dem Client-Computer übertragen werden. Bei der nächsten Server-Client-Session werden diese Daten dann an die zentrale Datenbank des Server-Computers übertragen, d.h. der Client nimmt Verbindung mit dem Server auf, um die Daten an die zentrale Datenbank zu übertragen.

Alternativ ist es auch möglich, das tragbare Eingabegerät mit einer Kommunikationsschnittstelle auszurüsten, welche unmittelbar die Synchronisation mit der zentralen Server-Datenbank erlaubt. Die Übertragung der entsprechenden Daten erfolgt dann beispielsweise über ein Mobiltelefonnetzwerk oder einen anderen Datendienst.

Nach einer weiteren bevorzugten Ausführungsform der Erfindung ist die Schnittstelle zwischen dem Client-Computer des Benutzers und dem tragbaren Eingabegerät als sogenannte Docking Station ausgebildet. Die Docking Station hat ein einzelnes Bedienelement, welches zur Eingabe eines Synchronisationskommandos dient. Zur Synchronisation stellt der Patient das Eingabegerät in die Docking Station und betätigt bei eingeschaltetem Client-Computer das Bedienelement. Hierdurch wird ein Programm gestartet, welches automatisch die Synchronisation der Daten des Eingabegeräts und der Daten des Client-Computers vornimmt. Zu der Synchronisation gehört insbesondere die Übertragung der in dem Eingabegerät lokal gespeicherten tatsächlichen Betreuungsablauf-Profildaten sowie gegebenenfalls auch die Aktualisierung des individuellen Betreuungsablauf-Profils, wenn der Benutzer zwischenzeitlich eine Veränderung seines individuellen Betreuungsablauf-Profils vorgenommen hat.

Wenn das tragbare Eingabegerät mit einer Kommunikationsschnittstelle ausgerüstet ist, welche den unmittelbaren Aufbau einer Kommunikationsverbindung mit der Server-Datenbank erlaubt, kann durch Betätigung des Bedienelements auch die Synchronisation der Daten unmittelbar mit der Server-Datenbank erfolgen. In diesem Fall nimmt das tragbare Eingabegerät selbst die Funktion des Client-Computers wahr.

Alternativ oder zusätzlich kann die Synchronisation auch automatisch erfolgen, d. h. ohne dass die Betätigung des Bedienelements zur Einleitung der Synchronisation erforderlich ist. Zum Beispiel kann eine Synchronisation zu vorgegebenen Zeitpunkten oder innerhalb vorgegebener Zeitintervalle erfolgen, wobei für die Synchronisation jeweils zwischen dem tragbaren Eingabegerät und der Server-Datenbank eine Kommunikationsverbindung aufgebaut wird. Vorzugsweise erfolgt dies drahtlos, beispielsweise über ein Mobiltelefonnetzwerk oder einen anderen Datendienst.

In Weiteren werden bevorzugte Ausführungsformen der Erfindung mit Bezugnahme auf die Zeichnungen näher erläutert. Es zeigen:
- Figur 1: ein Flussdiagramm einer bevorzugten Ausführungsform der Erfindung zur Eingabe eines individuellen Betreuungsablauf-Profils,
- Figur 2: ein Flussdiagramm einer bevorzugten Ausführungsform der Erfindung zur Eingabe und Speicherung tatsächlicher Betreuungsablauf-Profildaten,
- Figur 3: ein Flussdiagramm einer Ausführungsform der Erfindung für die Analyse von tatsächlichen Betreuungsablauf-Profildaten,
- Figur 4: eine Ausführungsform einer Eingabemaske für die Eingabe von Anamnesedaten,
- Figur 5: eine weitere Ausführungsform einer Eingabemaske für die Eingabe von Anamnesedaten,
- Figur 6: eine weitere Ausführungsform einer Eingabemaske für Anamnesedaten,
- Figur 7: eine Ausführungsform eines ersten individuellen Betreuungsablauf-Profils,
- Figur 8: einen Ausschnitt aus einem typischen Betreuungsablauf-Profil mit Standardvorgaben für die Einnahme verschiedener Mahlzeiten,
- Figur 9: ein weiteres typisches Betreuungsablauf-Profil,
- Figur 10: ein weiteres typisches Betreuungsablauf-Profil,
- Figur 11: eine Eingabemaske für die Ergänzung eines typischen Betreuungsablauf-Profils um Bewegungsdaten,
- Figur 12: eine erste Eingabemaske für die Eingabe von tatsächlichen Betreuungsablauf-Profildaten,
- Figur 13: eine zweite Eingabemaske für die Eingabe von tatsächlichen Betreuungsablauf-Profildaten,
- Figur 14: eine weitere Eingabemaske für die Eingabe von tatsächlichen Betreuungsablauf-Profildaten,
- Figur 15: eine Darstellung eines Blutzucker-Profils, basierend auf tatsächlichen Betreuungsablauf-Profildaten,
- Figur 16: eine Ausführungsform eines erfindungsgemäßen Computersystems,
- Figur 17: ein Verfahren zum Betrieb des Computersystem der Figur 16.

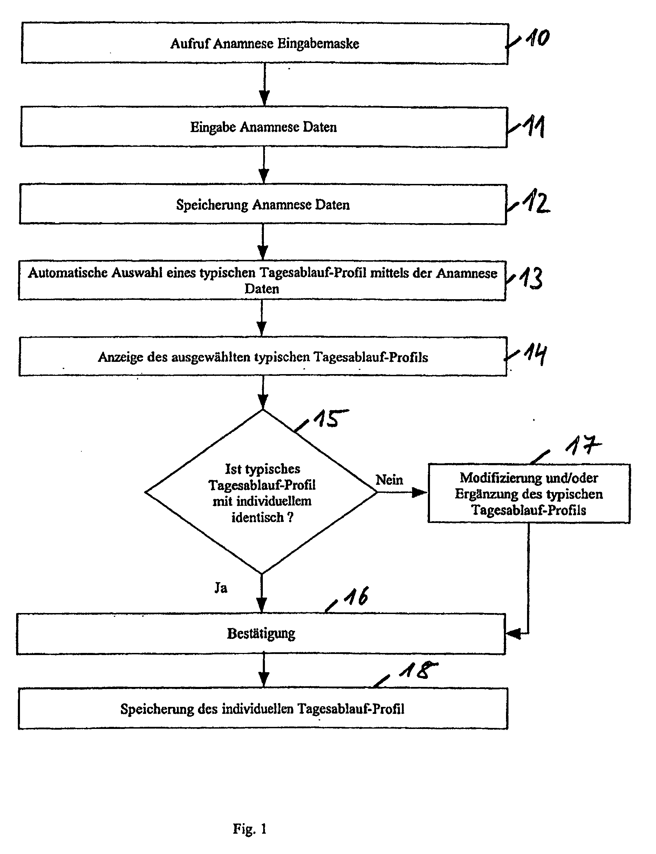

Die Figur 1 zeigt eine Ausführungsform eines erfindungsgemäßen Verfahrens für die Initialisierung der Teilnahme eines Patienten an einem Gesundheitsmanagementprogramm. Hierzu stellt der Benutzer zunächst eine Intemetverbindung mittels des Browser-Programms seines Client-Computers her. Bei dem Client-Computer kann es sich um einen üblichen Personal Computer handeln. Der Patient gibt die Web-Adresse der Gesundheitsmanagement-Webseite ein. Dort selektiert er beispielsweise durch Anklicken mit seiner Computermaus die Rubrik "Gesundheitsprogramme", um seinem Wunsch für die Teilnahme an einem Gesundheitsprogramm Ausdruck zu verleihen. Hierdurch wird in dem Schritt 10 eine Eingabemaske für die Eingabe von Anamnesedaten aufgerufen.

Vorzugsweise beinhaltet die Eingabemaske für die Anamnesedaten neben Daten bezüglich des Krankheitsverlaufs, der Diagnose und der Therapieform noch weitere personenbezogene Daten, wie z. B. die Adresse, Telefonnummer und E-Mail-Adresse des Patienten. Femer kann die Eingabemaske auch Eingabefelder für Angaben betreffend den behandelnden Arzt, insbesondere Name, Adresse, Telefonnummer und E-Mail-Adresse des behandelnden Arztes beinhalten.

In dem Schritt 11 gibt der Patient die entsprechenden Daten in die Eingabemaske für die Anamnese ein. Nach Beendigung der Eingabe betätigt der Patient mit seiner Computermaus ein virtuelles Bedienelement, das heißt, einen sogenannten Button, z. B. durch Anklicken auf der Eingabemaske, um die Eingabe der Anamnesedaten zu bestätigen. In dem Schritt 12 werden diese Anamnesedaten daraufhin zu dem Server-Computer des Gesundheitsmanagementprogramms übertragen und dort in einer Datenbank gespeichert.

Aufgrund der Speicherung der Anamnesedaten auf dem Server-Computer wird ein Programmmodul des Server-Computers gestartet, welches zur automatischen Auswahl eines typischen Betreuungsablauf-Profils entsprechend der Anamnese dient. Dies kann so realisiert sein, dass auf dem Server eine Datenbank mit typischen Betreuungsablaufprofilen vorhanden ist, wobei mit ein oder mehreren Anamnesedaten als Schlüssel auf ein typisches Betreuungsablauf-Profil zugegriffen werden kann.

Beispielsweise ist für eine bestimmte chronische Erkrankung und für eine bestimmte Therapieform dieser chronischen Erkrankung ein typisches Betreuungsablauf-Profil in der Datenbank vorhanden. Die in der Anamnese eingegebene Art der chronischen Erkrankung und die in der Anamneseeingabemaske eingegebene Therapieform dient dann zu Auswahl und zum Zugriff auf das entsprechende typische Betreuungsablauf-Profil. Alternativ können auch weitere Anamnesedaten für die Auswahl eines am besten passenden typischen Betreuungsablauf-Profil ausgewertet werden.

In dem Schritt 14 wird eine Eingabemaske mit dem ausgewählten typischen Betreuungsablauf-Profil zu dem Client-Computer des Benutzers übertragen und auf dessen Browser angezeigt. Die entsprechende Eingabemaske beinhaltet Eingabefelder für verhaltensbezogene Daten des Patienten und für die Eingabe von zumindest einem charakteristischen Messwert hinsichtlich der chronischen Erkrankung, also z. B. von einem oder mehren physiologischen Parametern. Die Eingabefelder für die verhaltensbezogenen Daten können dabei die Einnahme von Medikamenten zu bestimmten Uhrzeiten, die Aufnahme von Nahrung einer bestimmten Menge zu bestimmten Mahlzeiten, Art und Umfang einer körperlichen Betätigung zu einem bestimmten Zeitpunkt und / oder weitere verhaltenspezifische Daten des Patienten, welche für seine chronische Erkrankung relevant sind, sein.

Die Eingabefelder für die verhaltensbezogenen Daten des Patienten sind mit Standardeintragungen vorbelegt. Diese Standardeintragungen sind durch den Patienten editierbar, um sie auf die tatsächlichen Lebensgewohnheiten und die tatsächliche Behandlung des Patienten anzupassen. Ferner können in der Eingabemaske des typischen Betreuungsablauf-Profils auch weitere Eingabefelder vorhanden sein, die z. B. optional sind und für die keine Standardeintragung angegeben wird.

In dem Schritt 15 prüft der Patient, ob das typische Betreuungsablaufprofil, welches ihm in der angezeigten Eingabemaske angeboten wird, mit seinem individuellem Betreuungsablauf-Profil identisch ist. Wenn dies der Fall ist, das heißt, wenn sämtliche verhaltensbezogenen Standardeintragungen dem normalen oder angestrebten Betreuungsablauf-Profil des Patienten entsprechen, bestätigt der Patient im dem Schritt 16 durch Betätigung eines Bedienelements auf der Eingabemaske die Standardeintragungen des typischen Betreuungsablaufprofil.

Wenn es dagegen Abweichungen von dem typischen Betreuungsablaufprofil gibt, so gibt der Patient in dem Schritt 17 Daten zur Modifizierung und / oder Ergänzung des typischen Betreuungsablaufprofils ein. Dies kann durch Editieren der Standardeintragungen erfolgen und / oder durch Hinzufügung weiterer verhaltensbezogener Daten. Danach bestätigt der Patient in dem Schritt 16 wiederum die Eingabe.

Daraufhin werden die in der Eingabemaske vorhandenen Daten zu dem Server-Computer in dem Schritt 18 übertragen und als individuelles Betreuungsablauf-Profil des betreffenden Patienten in einer Datenbank gespeichert. Damit ist die Registrierung des Patienten als Nutzer des Gesundheitsmanagementprogramms und die Initialisierung für die Teilnahme an einem solchen Programm abgeschlossen. Von besonderem Vorteil ist dabei, dass der Patient nicht sämtliche verhaltensbezogenen Betreuungsablauf-Profildaten von Hand in die Eingabemaske des Schritts 14 eingeben muss.

Bei Übereinstimmung des typischen Betreuungsablauf-Profils mit dem individuellen Betreuungsablauf-Profil des Patienten ist lediglich eine Bestätigung erforderlich. Bei Abweichungen von dem typischen Betreuungsablauf-Profil ist lediglich die Eingabe derjenigen Daten erforderlich, hinsichtlich derer sich das typische Betreuungsablauf-Profil von dem individuellen Betreuungsablauf-Profil des Patienten unterscheidet. Dies stellt eine wesentliche Erleichterung für den Patienten dar und senkt entsprechend die Schwelle für die Teilnahme an dem Gesundheitsmanagementprogramm.

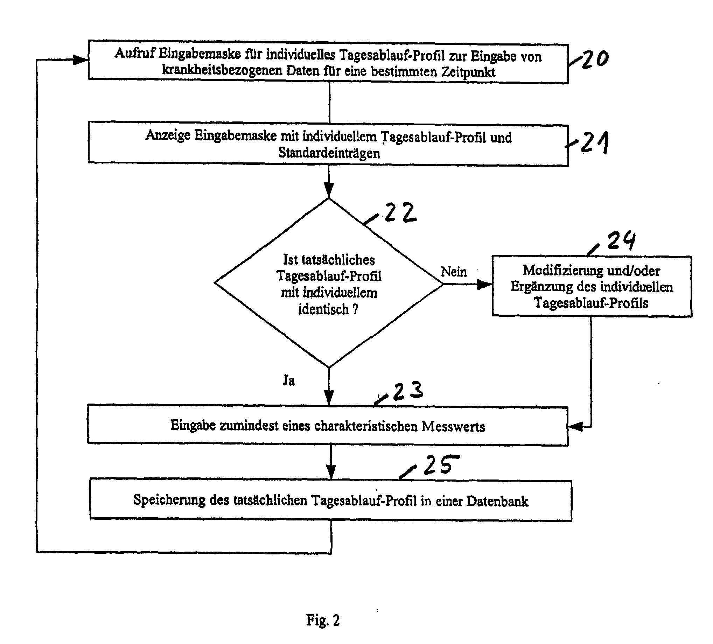

Die Figur 2 zeigt ein Flussdiagramm hinsichtlich der fortlaufenden Datenerfassung für die Ermittlung des Verlaufs der chronischen Erkrankung des Patienten. Für die Datenerfassung baut der Patient zunächst eine Internet-Verbindung zwischen seinem Client-Computer und dem Server-Computer des Gesundheitsmanagementprogramms auf. Dort muss sich der Benutzer vorzugsweise zunächst authentifizieren, z. B. durch Eingabe seiner Benutzer-ID und seines Passworts. Der Server-Computer greift dann z. B. mit der Benutzer-ID als Schlüssel auf das individuelle Betreuungsablauf-Profil des Benutzers zu, welcher dieser zuvor gemäß einem Verfahren, wie mit Bezug auf die Figur 1 erläutert, spezifiziert hat.

Beispielsweise durch "Anklicken" eines virtuellen Bedienelements auf der Home Web-Seite des Gesundheitsmanagementprogramms ruft der Patient in dem Schritt 20 eine Eingabemaske auf, welche seinem individuellem Betreuungsablauf-Profil entspricht. Dies bedeutet, dass die Eingabemaske bereits in einem oder mehreren der Datenfelder für die Eingabe von verhaltensbezogenen Daten Eintragungen beinhaltet, die sich aus dem individuellen Betreuungsablauf-Profil ergeben. Die Anzeige dieser Eingabemaske mit dem individuellen Betreuungsablauf-Profil und den entsprechenden individualisierten Standardeintragungen des Patienten erfolgt in dem Schritt 21.

In dem Schritt 22 prüft der Patient, ob sein individuelles Betreuungsablauf-Profil gemäß Eingabemaske mit dem tatsächlichen Betreuungsablauf-Profil übereinstimmt. Dies bedeutet, dass der Benutzer für die einzelnen Standardeintragungen überprüft, ob er sich tatsächlich so, wie geplant verhalten hat, oder ob eine Abweichung vorliegt.

Wenn eine solche Abweichung vorliegt, gibt der Benutzer in dem Schritt 24 entsprechende Daten zur Modifizierung und / oder Ergänzung des individuellen Betreuungsablauf-Profils ein, das heißt, der Benutzer editiert beispielsweise seine individualisierten Standardeintragungen und / oder er gibt zusätzliche Informationen ein.

In dem nachfolgenden Schritt 23 ermittelt der Patient einen charakteristischen Messwert, der für die Beurteilung des Verlaufs seiner chronischen Erkrankung relevant ist. Hierbei kann es sich je nach der Art der chronischen Erkrankung um einen physiologischen Messwert, wie z. B. den Blutzuckerspiegel im Fall von Diabetes oder den sogenannten Peak-Flow im Fall von Asthma handeln.

Diesen Messwert gibt der Patient in dem Schritt 23 in die Eingabemaske seines individuellen Betreuungsablauf-Profils ein.

Wenn die Prüfung in dem Schritt 22 ergeben hat, dass das tatsächliche Tageablauf-Profil mit dem individuellen Betreuungsablauf-Profil übereinstimmt, kann der Patient gleich zu dem Schritt 23 übergehen und die Eingabe des charakteristischen Messwerts vornehmen.

Danach bestätigt der Patient die Daten in den Eingabefeldern der Eingabemaske seines individuellen Betreuungsablauf-Profils, nachdem diese gegebenenfalls modifiziert oder ergänzt (Schritt 24) worden sind und nachdem der charakteristische Messwert in die Eingabemaske eingetragen worden ist. Hierdurch werden die entsprechenden Eingabedaten, dass heißt die tatsächlichen Betreuungsablauf-Profildaten über eine Kommunikationsverbindung, z. B. das Internet, zu dem Server-Computer übertragen und dort in dem Schritt 25 in einer Datenbank abgespeichert.

Danach kann der Patient erneut in dem Schritt 20 eine Eingabemaske aufrufen. Der Patient kann diese Art der Eingabe mehrfach täglich durchführen und so während des Betreuungsablaufs die tatsächlichen Betreuungsablauf-Profildaten schrittweise ergänzen. Der Patient kann jedoch auch nur innerhalb größerer Abstände entsprechende Eingaben vornehmen, insbesondere wenn die Art der Erkrankung nicht so schwerwiegend ist, dass eine tägliche Überwachung des Verlaufs der chronischen Erkrankung erforderlich ist. Entsprechend kann es sich dann an der Stelle von Betreuungsablauf-Profildaten auch um größere Zeiträume, wie z. B. ein Woche oder einen Monat handeln.

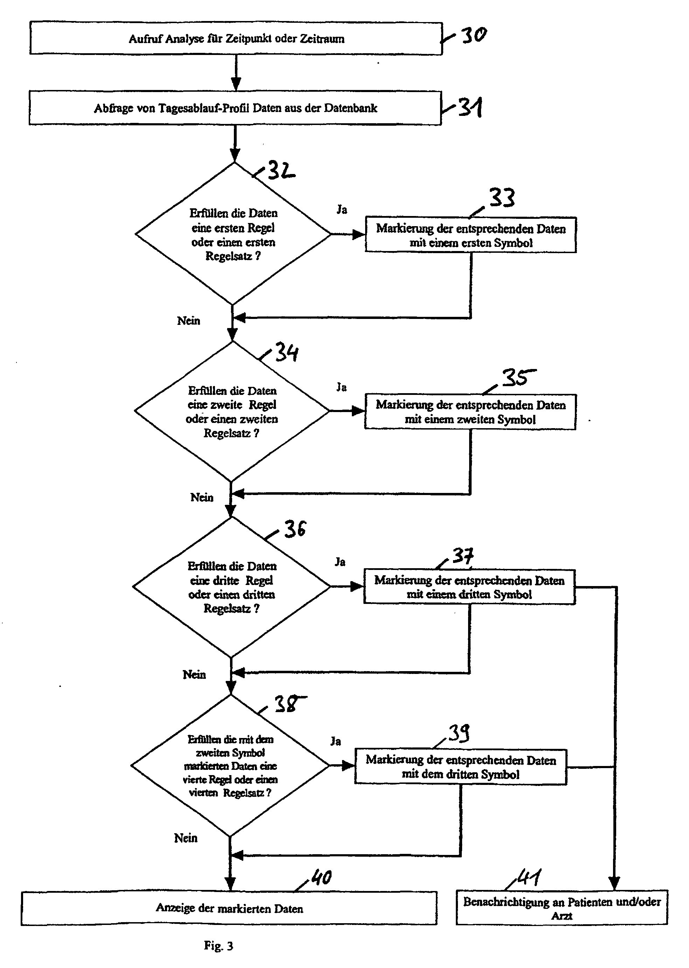

Die Figur 3 zeigt ein Flussdiagramm für die Analyse der tatsächlichen Betreuungsablauf-Profildaten (z.B. den Verlauf relevanter medizinischer oder administrativer Parameter) eines Patienten. Vorzugsweise erfolgt diese Analyse serverseitig automatisch oder nach einer entsprechenden Nutzeranforderung.

In dem Schritt 30 wird das entsprechende Programmmodul für die Analyse auf dem Server-Computer aufgerufen. Bei diesem Aufruf kann als Parameter der Zeitpunkt oder der Zeitraum für die Analyse spezifiziert werden.

In dem Schritt 31 werden die tatsächlichen Betreuungsablauf-Profildaten des betreffenden Patienten für den Zeitpunkt oder den Zeitraum aus der Datenbank abgerufen.

In dem Schritt 32 überprüft das Programmmodul, ob diese Daten eine bestimmte Regel oder einen bestimmten Regelsatz erfüllen. Beispielsweise kann es sich bei einer solchen Regel um einen Überprüfung handeln, ob ein bestimmter charakteristischer Messwert in einem bestimmten Normalbereich liegt. Wenn dies der Fall ist, wird der entsprechende Messwert mit einem ersten Symbol markiert. Dies kann beispielsweise so erfolgen, dass der betreffende Messwert in einer Ausgabemaske der Analyse grün hinterlegt angezeigt wird.

Ist das Gegenteil der Fall, das heißt, liegen eine oder mehrere der Daten nicht in dem Normbereich, so wird in dem Schritt 34 geprüft, ob die Daten eine weitere Regel oder einen weiteren Regelsatz erfüllen. Dies kann beispielsweise so erfolgen, dass geprüft wird, ob ein bestimmter Messwert innerhalb eines Grenzbereichs liegt. Der Grenzbereich zeigt eine gewisse Risikosituation an, die jedoch für sich gesehen zunächst nicht akut oder bedrohlich ist. Wenn ein bestimmter Messerwert in einem solchen Grenzbereich liegt, wird der entsprechende Wert in dem Schritt 35 mit einem zweiten Symbol markiert, beispielsweise indem der betreffende Messwert in der Ausgabemaske der Analyse gelb hinterlegt angezeigt wird.

Nachfolgend wird in dem Schritt 36 geprüft, ob die Betreuungsablauf-Profildaten eine weitere Regel oder einen weiteren Regelsatz erfüllen. Hierbei kann es sich beispielweise um die Prüfung handeln, ob ein bestimmter Messwert in einem Risikobereich liegt, der ein unmittelbares und akutes Gesundheitsrisiko für den Patienten darstellt. Wird eine solche Regel oder ein solcher Regelsatz erfüllt, so wird der oder die betreffenden Messwerte mit einem dritten Symbol markiert, beispielsweise durch eine rote Hinterlegung. Dies erfolgt in dem Schritt 37.

Danach wird in dem Schritt 38 geprüft, ob die in dem Schritt 35 mit dem zweiten Symbol markierten Daten eine vierte Regel oder einen vierten Regelsatz erfüllen. In dem Schritt 38 wird also geprüft, ob die Kombination der als grenzwertig identifizierten Messwerte ein besonderes Risiko darstellt. Beispielsweise gibt es medizinische Situationen, in denen zwei verschiedene Messwerte jeweils in einem Grenzbereich liegen, der für sich gesehen noch unkritisch ist, wobei jedoch die Kombination der beiden grenzwertigen Messwerte eine Risikosituation ergibt. Wenn eine solche Regel erfüllt wird, werden die betreffenden Daten daher in dem Schritt 39 ebenfalls mit dem dritten Symbol markiert, wobei des zweite Symbol entweder beibehalten oder aufgehoben wird. Beispielsweise werden also die zuvor mit gelben Hintergrund markierten Daten mit einem roten Hintergrund markiert.

In dem Schritt 40 erfolgt dann eine Anzeige der markierten Daten in dem Ausgabefenster der Analyse. Femer erfolgt gegebenenfalls in dem Schritt 41 eine automatische Benachrichtigung des Patienten und / oder des behandelnden Arztes oder eines anderen Gesundheitsdienstleisters, wobei die entsprechende Nachricht einen Hinweis auf das Vorliegen einer akuten Risikosituation beinhaltet. Diese Benachrichtigung wird automatisch von dem Serversystem generiert und kann als elektronische Mitteilung, z. B. per E-Mail, SMS oder als Fax versendet werden. Ferner kann auch eine Übernahme der Benachrichtigung in das Reminder-System, die Generierung eines automatischen Anrufs oder eine andere geeignte Form der Informationsübermittlung an den zuständigen Betreuuer erfolgen.

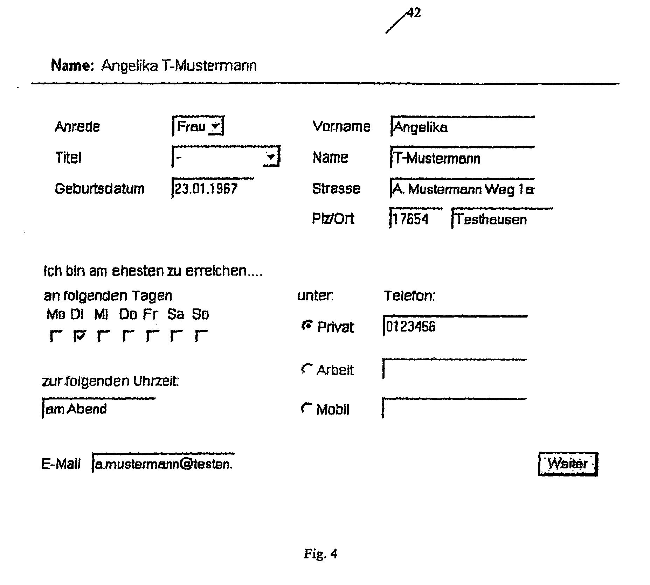

Die Figuren 4, 5 und 6 zeigen Eingabemasken für die Eingabe von Anamnesedaten (vgl. die Schritte 10 bis 12 der Figur 1).

Die Eingabemaske 42 der Figur 4 beinhaltet Datenfelder für die Eingabe von personenbezogenen Daten des Patienten, sowie dessen Anrede, Titel, Geburtsdatum, Vorname, Straße, Postleitzahl / Ort, Telefonnummer, Privat, in der Arbeitsstelle sowie die Mobiltelefonnummer und Eingabefelder zur Spezifizierung der Erreichbarkeit an bestimmten Tagen zu bestimmten Uhrzeiten. Ferner ist auch ein Eingabefeld für die Eingabe der E-Mail-Adresse des Patienten vorhanden. Die E-Mail Adresse ist insbesondere für die automatische Versendung einer Benachrichtigung an den Patienten (vgl. Schritt 41 der Figur 3) von Bedeutung.

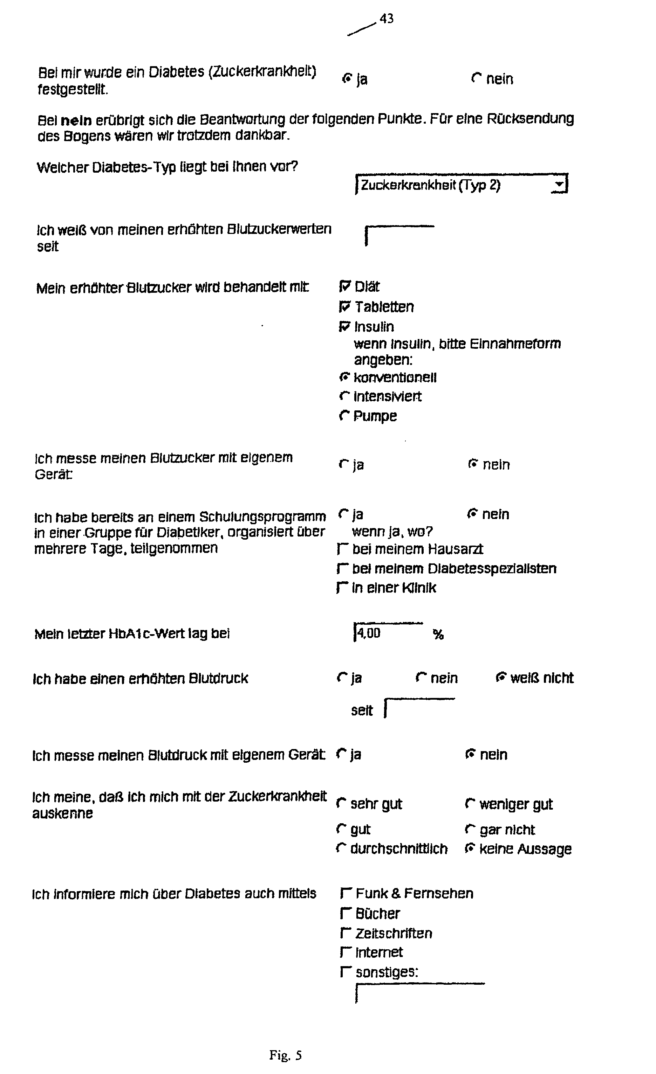

Die Eingabemaske 43 der Figur 5 beinhaltet weitere Eingabefelder, welche spezifisch für die chronische Erkrankung des Patienten sind. In diesem Fall handelt es sich um einen Diabetes-Patienten. Insbesondere sind Eingabefelder vorhanden für die Spezifizierung des Typs der Zuckerkrankheit und der Art der Behandlung der Zuckerkrankheit mittels einer Diät, Tabletten oder Insulin. Für Insulin ist ferner die Einnahmeform spezifizierbar, nämlich konventionell, intensiviert oder mittels einer Pumpe. Diese Angaben des Patienten hinsichtlich der Therapieform sind von besonderer Bedeutung für die automatische Auswahl eines typischen Betreuungsablauf-Profils für die betreffende Erkrankung (vgl. Schritt 13 der Figur 1).

Die Eingabemaske 43 beinhaltet weitere Eingabefelder, welche für die Typisierung der chronischen Erkrankung relevant sein können ("ich weiß von meinen erhöhten Blutzuckerwerten seit", "mein letzter HbA1c-Wert lag bei", "ich habe einen erhöhten Blutdruck"). Ferner sind Eingabefelder vorhanden, um den Patienten zu Typisieren ("ich habe bereits an einem Schulungsprogramm in einer Gruppe für Diabetiker, organisiert, über mehrere Tage, teilgenommen", "ich meine, dass ich mich mit der Zuckerkrankheit auskenne", "ich informiere mich über Diabetes auch mittels"). Des weiteren sind Eingabefelder vorhanden, um Daten betreffend der Geräte Ausstattung des Patienten einzugeben ("ich messe meinen Blutzucker mit eigenem Gerät").

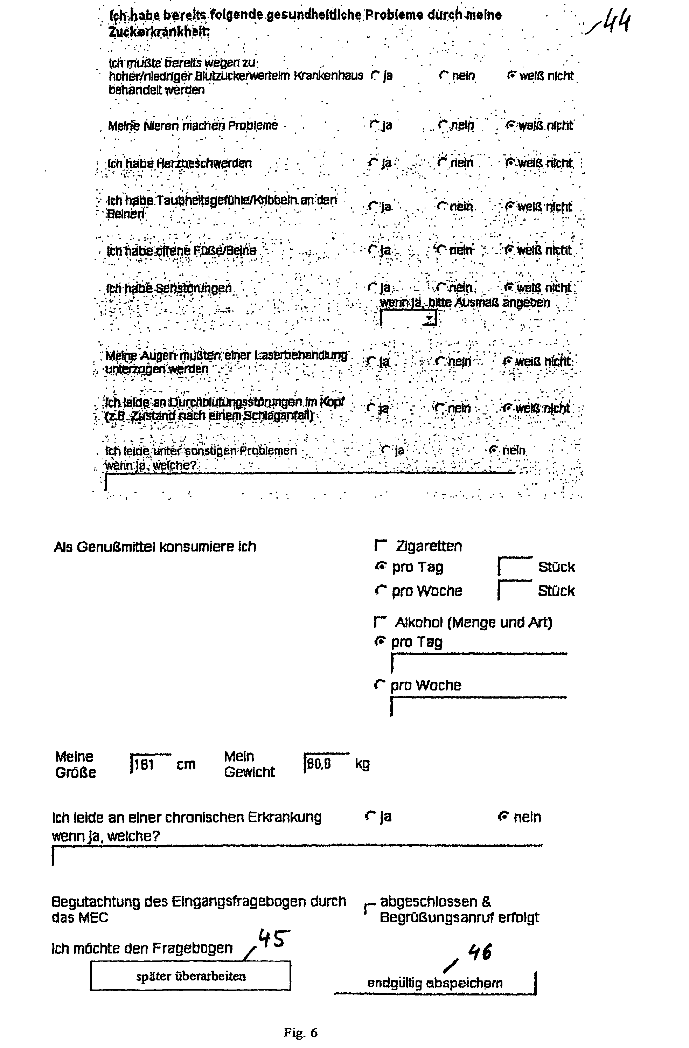

Die Figur 6 zeigt eine Eingabemaske 44 zur Eingabe weiterer krankheitsbezogener Daten ("ich habe bereits folgende gesundheitliche Probleme durch meine Zuckerkrankheit:") sowie verhaltensbezogene Daten ("als Genussmittel konsumiere ich ...") sowie zur Eingabe von Größe und Gewicht und zur Angabe einer weiteren chronischen Erkrankung.

Der Patient hat dann die Möglichkeit die in den Eingabemasken 42, 43 und 44 eingegebenen Daten später zu überarbeiten oder entgültig abzuspeichern, indem entweder das Bedienelement 45 oder das Bedienelement 46 betätigt wird.

Nach der Betätigung des Bedienelements 46 werden die Anamnesedaten an den Server-Computer übertragen und die Registrierung des Patienten ist abgeschlossen. Basierend auf den Anamnesedaten erfolgt dann serverseitig eine automatische Auswahl des typischen Betreuungsablauf-Profils des Patienten (vgl. Schritt 13 der Figur 1).

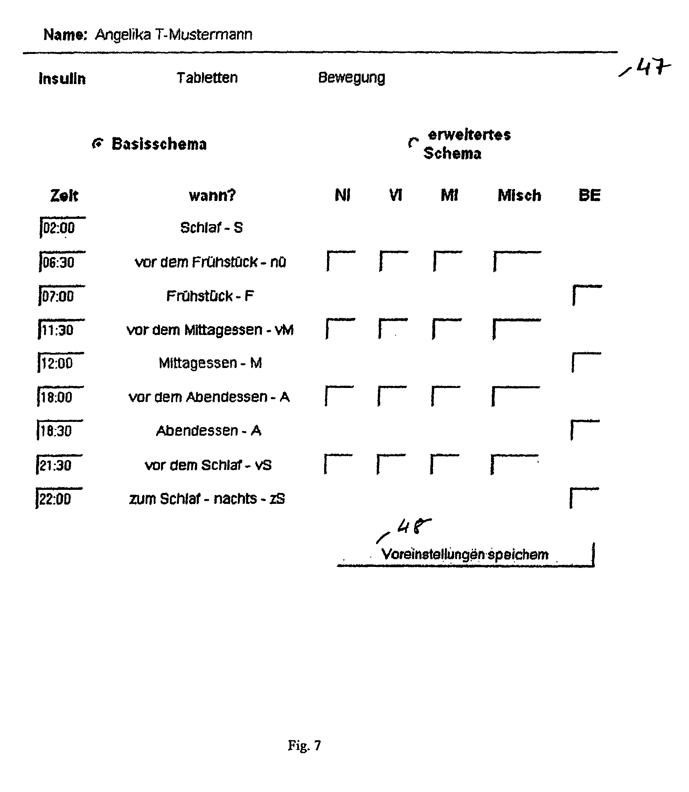

Die Figur 7 zeigt eine entsprechende Eingabemaske 47 für ein automatisch ausgewähltes typisches Betreuungsablauf-Profil, und zwar für die Therapieform "Insulin". In der Eingabemaske 47 sind dazu Eingabefelder vorhanden, um einzugeben, zu welchen Zeitpunkt der Patient Insulin appliziert oder Nahrung aufgenommen hat und wie viele Broteinheiten ("BE") er dabei zu sich nimmt. Ferner sind auch Dateneingabefelder für die verschiedenen Zeitpunkte vorhanden, um die Art des jeweils aufgenommenen Insulins zu spezifizieren ("NI", "VI", "MI", "Misch"). Die Eingabefelder für die verschiedenen Zeiten sind dabei mit Standardeintragungen vorbelegt, die editierbar sind. Von Besonderheit ist dabei, dass die Zeitpunkte an den Tagesrhytmus angepasst sind und nicht ausschließlich über die Uhrzeiten definiert werden.

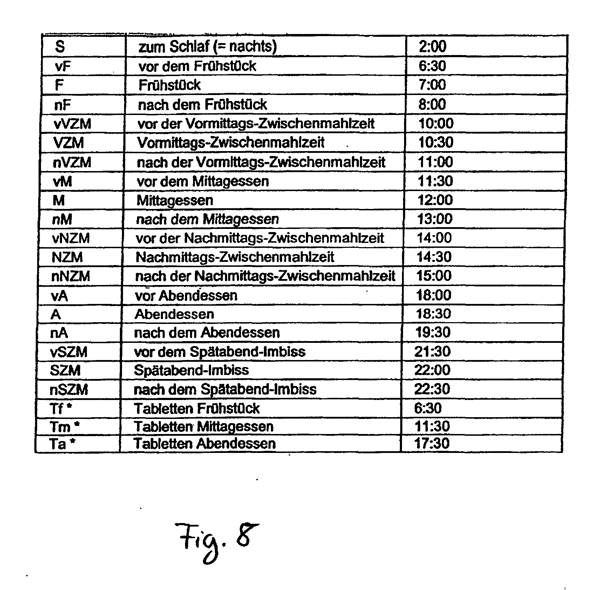

Die Figur 8 zeigt eine Tabelle, die die Standardeintragungen von Uhrzeiten für die verschiedenen Mahlzeiten für eine bestimmte Therapieform typisiert angibt.

Von besonderem Vorteil ist dabei, dass die Eingabemaske 47 der Figur 7 unmittelbar auf die Art der Therapie bezogen ist, indem sie die relevanten Zeitpunkte und die Medikamentierung vorgibt. Darüber hinaus können auch z.B. die Aufnahme von Nahrungsmitteln in einer bestimmten Menge zu bestimmten Zeitpunkten vorgegeben sein.

Dieses vorgegebene Schema kann dem individuellen Betreuungsablauf-Profil des Patienten entsprechen. Es kann jedoch auch individuelle Abweichungen geben, die der Patient z.B. durch Editieren der vorgegebenen Zeiten eingeben kann. Nachdem der Patient alle erforderlichen Eingaben vorgenommen hat, betätigt er das Bedienelement 48, um diese Voreinstellungen zu speichern.

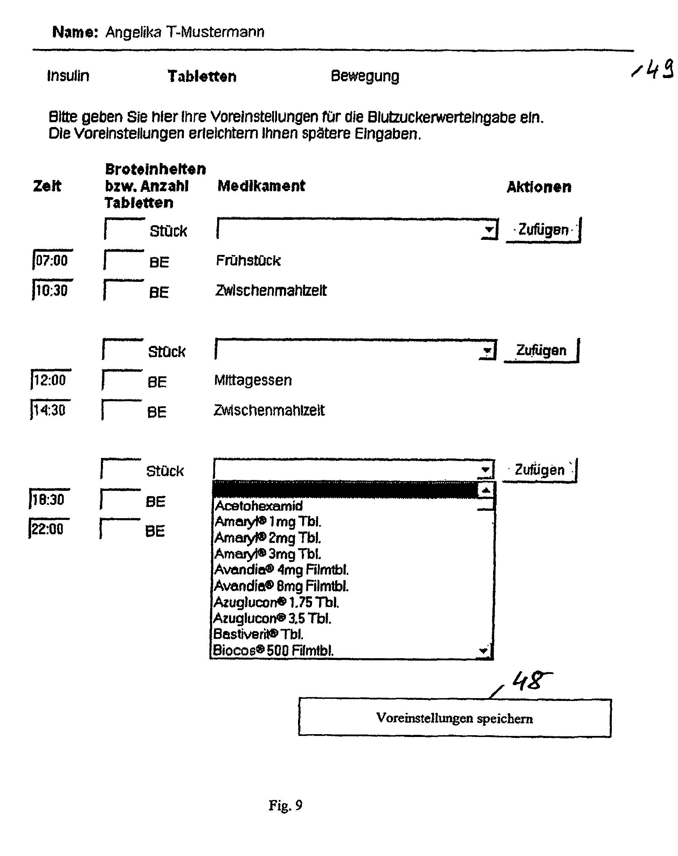

Die Figur 9 zeigt eine Eingabemaske 49 für den Fall der Auswahl eines typischen Betreuungsablauf-Profils für die Therapieform "Tabletten". Die Eingabemaske 49 ist wiederum direkt auf diese Therapieform bezogen und beinhaltet nur die hierfür relevanten Eingabefelder, wobei die Zeitpunkte und die Anzahl der Tabletten mit Eintragungen vorbelegt sind. Diese sind wiederum editierbar.

Die Eingabemaske 50 gibt schließlich ein typisches Betreuungsablauf-Profil für den Fall der Therapieform "Diät" an. Für diese Therapieform ist lediglich die Anzahl der von dem Patienten aufgenommenen Broteinheiten zu den verschiedenen Mahlzeiten relevant. Entsprechend fehlen hier Eingabefelder für die Medikamentierung.

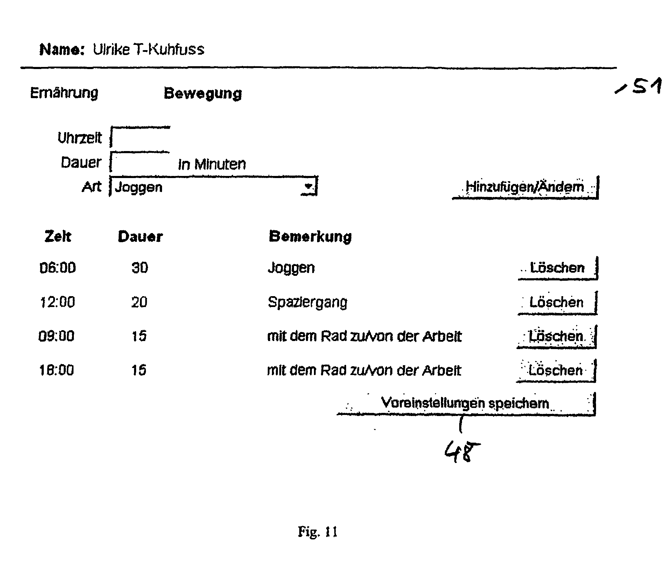

Die Eingabemaske 51 der Figur 11 ist eine optionale Eingabemaske, die es dem Patienten erlaubt, individuell einen Stundenplan für körperliche Bewegung und Sport einzugeben. Die Daten der Eingabemasken 47, 49 bzw. 50 sowie optional der Eingabemaske 51 werden dann als individuelles Betreuungsablauf-Profil nach Betätigung des Bedienelements 48 zu dem Server übertragen und dort gespeichert (vgl. Schritt 18 der Figur 1).

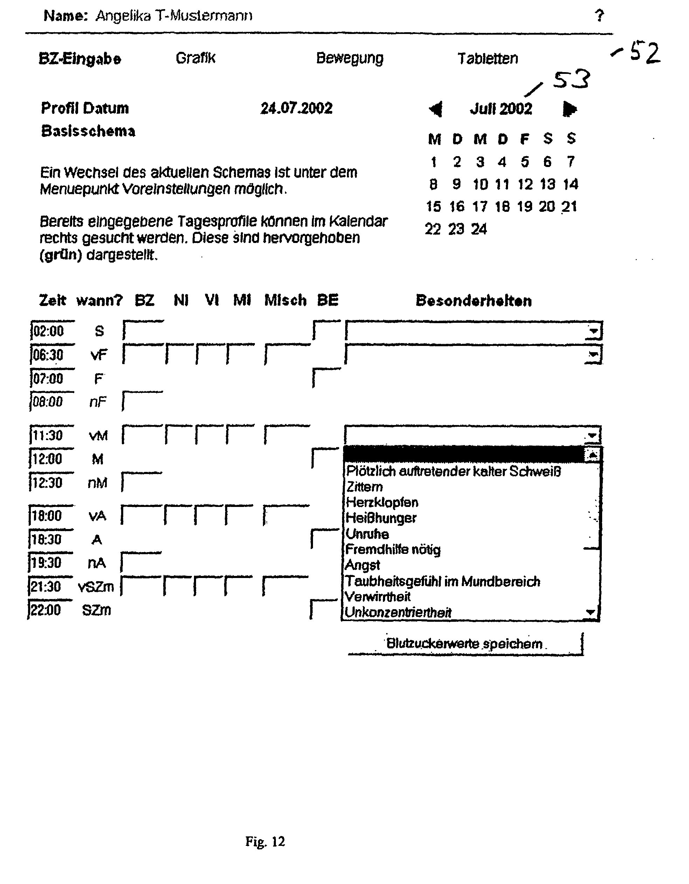

Die Eingabemasken der Figuren 12, 13 und 14 dienen zur Eingabe eines tatsächlichen Betreuungsablauf-Profils basierend auf dem individuellem Betreuungsablauf-Profil eines Patienten (vgl. Figur 2).

Die Figur 12 zeigt eine Eingabemaske 52 basierend auf dem individuellem Betreuungsablauf-Profil der Eingabemaske 47. Durch Anklicken eines bestimmten Datums auf dem Kalenderfeld 53 erhält der Patient eine Eingabemaske für das betreffende Datum mit individualisierten Standardeintragungen, die der Benutzer editieren und vervollständigen kann.

Der Patient kann zu dieser Eingabemaske 52 beispielsweise folgenden Hilfe-Text abrufen:

### Profil Basisschema

In dieser Maske haben Sie die Möglichkeit, Ihre Blutzuckerwerte in Verbindung mit der Uhrzeit bzw. Tageszeit (z.B. vor dem Abendessen) und Ihrer Insulintherapie einzugeben. Sollten Sie in der Voreinstellung, d.h. in Ihrem individuellen Betreuungsablauf-Profil, die Einnahme Ihrer Broteinheiten (Berechnungseinheiten, Kohlenhydrateinheiten, ...) sowie Ihrer Insulintherapie eingegeben und gespeichert haben, so werden diese automatisch in diese Maske hier übertragen. Tagesspezifische Änderungen (d.h. für die Eingabe des tatsächlichen Betreuungsablauf-Profils) können natürlich in der Maske "Blutzuckerwerteingabe" (Eingabemaske 52) vorgenommen werden, dauerhafte Änderungen sollten Sie in der Maske "Voreinstellungen" (d.h. in Ihrem individuellen Betreuungsablauf-Profil - Eingabemaske 47) abspeichern.

Datum: Im Feld erscheint automatisch das aktuelle Datum. Änderungen im betreffenden Monat können Sie durch Anklicken eines bestimmten Tages vornehmen. Durch Anklicken des Symbol "<" können Sie einen Monat zurück blättern, durch Anklicken des Symbol ">" einen Monat vorwärts blättern. Der durch Sie neu bestimmte Tag wird automatisch in das Datumsfeld übernommen.

Zeit: Im Feld erscheint die Eingabe der Voreinstellung, sofern Sie dort einen Eintrag getätigt haben. Sie können hier tagesspezifische Änderungen oder Neueinträge für die Uhrzeit (z. B. 12:00) eingeben, zu welcher Sie den Blutzucker kontrolliert haben, Ihr Insulin injizieren, die Mahlzeiten einnehmen bzw. Besonderheiten aufgetreten sind.

wann?: S = zum Schlaf, nachts; vF = vor dem Frühstück; F = Frühstück; nF = nach dem Frühstück; vM = vor dem Mittagessen; M = Mittagessen; nM = nach dem Mittagessen; vA = vor dem Abendessen; A = Abendessen; nA = nach dem Abendessen; vSZm = vor der Spätmahlzeit; SZm = Spätmahlzeit

BZ: Hier können Sie Ihren Blutzuckerwert (z. B. 137) eingeben, welcher zur voran angegebenen Uhrzeit bzw. Tageszeit, kontrolliert wurde. Es stehen Ihnen maximal acht Eingabemöglichkeiten zur Verfügung.

NI: Im Feld erscheint die Eingabe der Voreinstellung, sofern Sie dort einen Eintrag getätigt haben. Sie können hier tagesspezifische Änderungen oder Neueinträge für die Menge der Insulineinheiten (z. B. 10) eingeben, sofern Sie ein kurz wirkendes Normalinsulin oder ein schnell wirkendes Analoginsulin (z.B. Humalog, Novo Rapid) injizieren. Es stehen Ihnen maximal vier Eingabemöglichkeiten zur Verfügung.

VI: Im Feld erscheint die Eingabe der Voreinstellung, sofern Sie dort einen Eintrag getätigt haben. Sie können hier tagesspezifische Änderungen oder Neueinträge für die Menge der Insulineinheiten (z. B. 15) eingeben, sofern Sie ein lang wirkendes Verzögerungsinsulin injizieren. Es stehen Ihnen maximal vier Eingabemöglichkeiten zur Verfügung.

MI: Im Feld erscheint die Eingabe der Voreinstellung, sofern Sie dort einen Eintrag getätigt haben. Sie können hier tagesspezifische Änderungen oder Neueinträge für die Menge der Insulineinheiten (z. B. 20) eingeben, sofern Sie ein Misch- oder Kombinationsinsulin injizieren. Es stehen Ihnen maximal vier Eingabemöglichkeiten zur Verfügung.

Misch: Im Feld erscheint die Eingabe der Voreinstellung, sofern Sie dort einen Eintrag getätigt haben. Sie können hier tagesspezifische Änderungen oder Neueinträge für das Mischungsverhältnis (z. B. 30/70) eingeben, sofern Sie ein Misch- oder Kombinationsinsulin injizieren. Die erste Zahl beziffert den Anteil des kurz wirkenden Normalinsulin bzw. des schnell wirkenden Analoginsulin. Die zweite Zahl beziffert den Anteil des lang wirkenden Verzögerungsinsulin (z.B. 30/70). Es stehen Ihnen maximal vier Eingabemöglichkeiten zur Verfügung.

BE: Im Feld erscheint die Eingabe der Voreinstellung, sofern Sie dort einen Eintrag getätigt haben. Sie können hier tagesspezifische Änderungen oder Neueinträge für die Anzahl (z. B. 3) der Broteinheiten (Berechnungseinheiten, Kohlenhydrateinheiten, ...) eintragen. Es stehen Ihnen maximal fünf Eingabemöglichkeiten zur Verfügung.

Besonderheiten: Nach Anklicken des Pfeils öffnet sich eine Liste mit Besonderheiten (z.B. Anzeichen einer Unterzuckerung), diese finden Sie in alphabetischer Reihenfolge. Durch Anklicken einer Besonderheit wird diese automatisch in das Feld übernommen. Es stehen Ihnen maximal fünf Eingabemöglichkeiten zur Verfügung.

Blutzuckerwerte speichern: Beim Anklicken des Buttons wird Ihre Blutzuckerwerteingabe gespeichert.

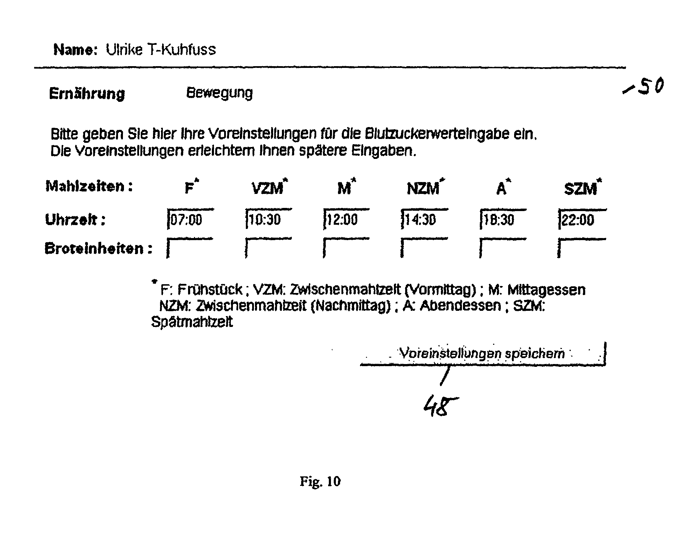

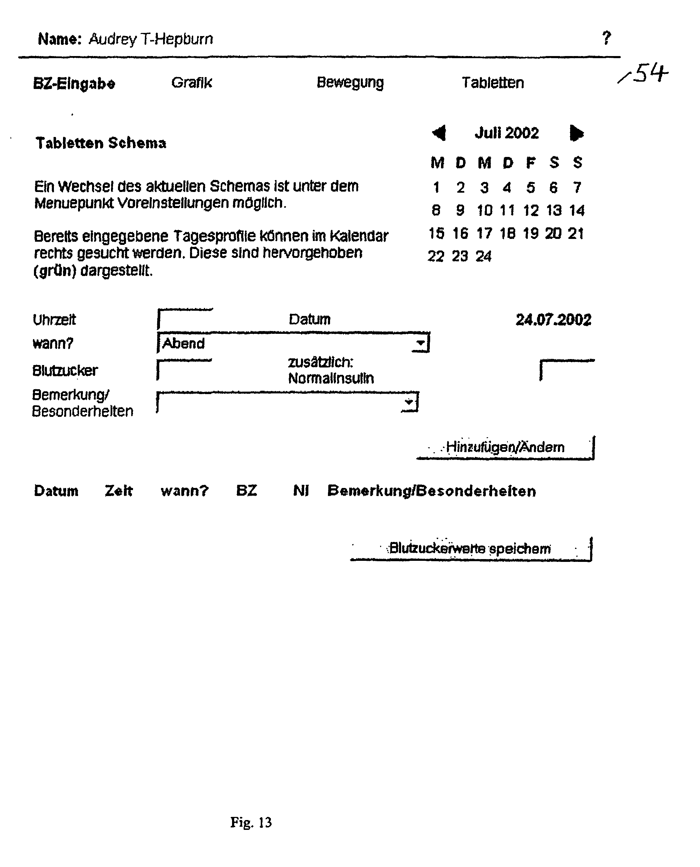

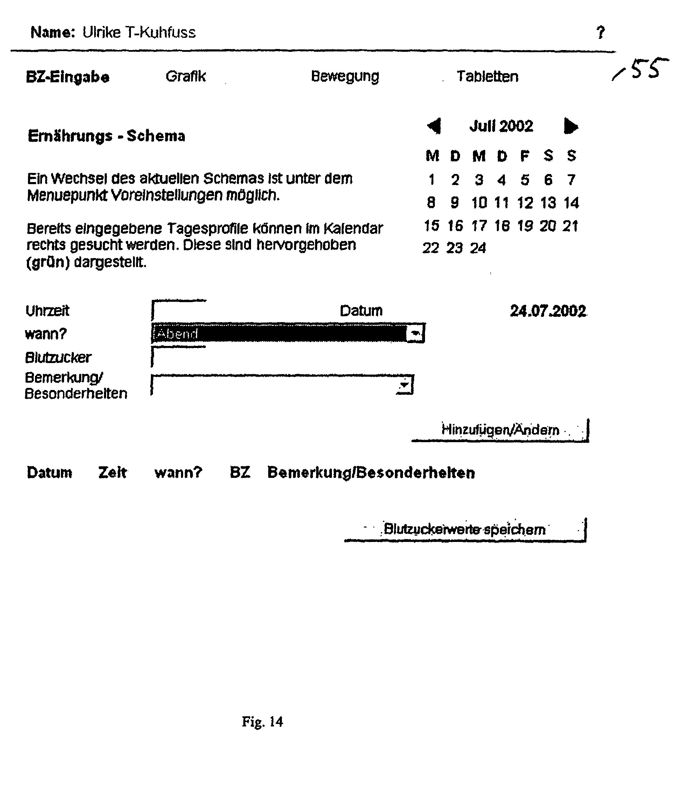

Die Eingabemaske 54 der Figur 13 bezieht sich auf das individuelle Betreuungsablauf-Profil gemäß der Eingabemaske 49. Die Eingabemaske 55 der Figur 14 bezieht sich auf die Eingabemaske 50 der Figur 10 für den Fall der diätischen Therapie.

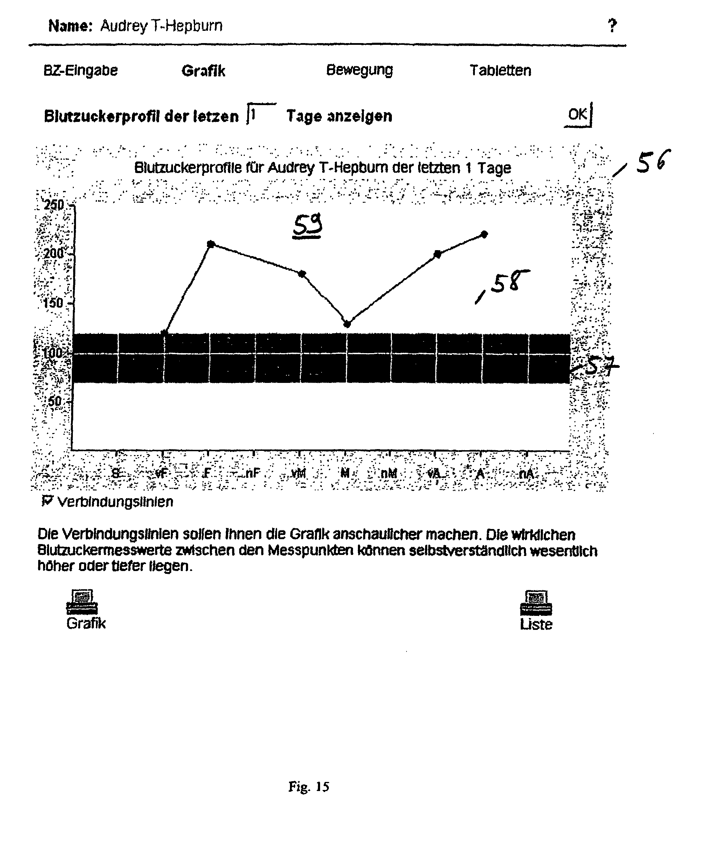

Wenn der Benutzer gemäß des Flussdiagramms der Figur 2 fortlaufend Eingaben vornimmt, resultieren so tatsächliche Betreuungsablauf-Profildaten für einen bestimmten Zeitraum. Diese können in einer Grafik gemäß Figur 15 dargestellt werden. Die Grafik 56 der Figur 15 zeigt das Blutzuckerprofil des letzten Tages für den fiktiven Patienten "Audrey T-Hepburn" jeweils zu bestimmten Zeitpunkten. Ebenfalls ist in der Grafik eingeblendet, ob es sich bei einem Blutzuckerwert um einen Wert innerhalb eines Normalbereichs 57, eines Grenzbereichs 58 oder eines Risikobereichs 59 handelt.

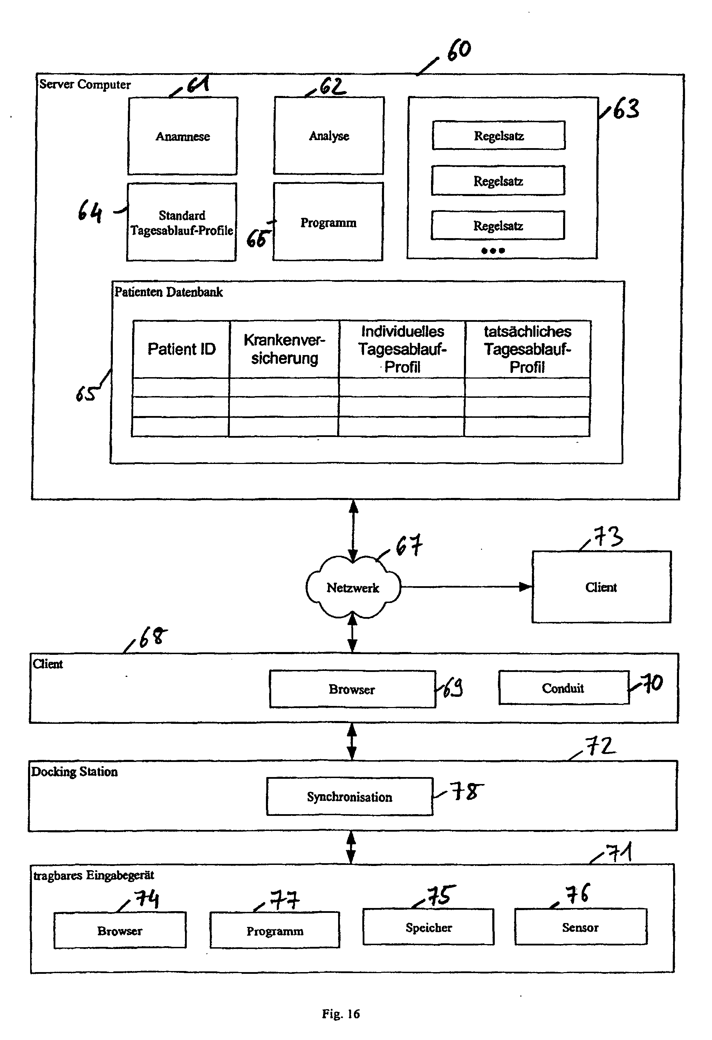

Die Figur 16 zeigt einen Server-Computer 60. Der Server-Computer 60 hat ein Anamnesemodul 61, ein Analysemodul 62, ein Regelsystem 63 mit verschiedenen Regelsätzen (vgl. Figur 3), eine Datenbank 64 zur Speicherung von typischen Betreuungsablauf-Profilen, eine Datenbank 65 zur Speicherung von patientenspezifischen Daten und ein Programm 66.

Die Datenbank 65 beinhaltet für jeden registrierten Patienten eine Benutzer-ID ("Patient-ID"). Der Benutzer-ID des Patienten ist dessen Krankenversicherungsnummer zugeordnet. Die Krankenversicherungsnummer dient zur Abrechnung der von dem Betreiber des Server-Computers 60 erbrachten Dienstleistungen gegenüber der Krankenversicherung des Patienten.

Ferner ist der Benutzer-ID das individuelle Betreuungsablauf-Profil des Patienten zugeordnet (vgl. Schritt 18 der Figur 1). Des weiteren sind der Benutzer-ID die tatsächlichen Betreuungsablauf-Profildaten zugeordnet (vgl. Schritt 25 der Figur 2).

Der Server-Computer 60 ist über ein Netzwerk 67, beispielsweise über das Internet mit einem Client-Computer 68 verbindbar. Bei dem Client-Computer 68 handelt es sich um einen üblichen Personal-Computer, den der Patient für die Kommunikation mit dem Server-Computer 60 benutzt. Der Client-Computer 68 hat ein übliches Browser-Programm 69, beispielsweise Netscape Navigator oder Microsoft Explorer. Ferner hat der Client-Computer 68 einen sogenannten Conduit 70 für die Synchronisation mit dem tragbaren Eingabegerät 71.

Ein Synchronisations-Modul, z. B. eine Docking Station 72 bildet das Interface zwischen dem Client-Computer 68 und dem tragbaren Eingabegerät 71.

Bei dem Client-Computer 73 handelt es sich um den Computer eines Dritten, z. B. des behandelnden Arztes.

Für die Benutzer-Registrierung und Anamnese stellt der Patient mittels des Browser-Programms 69 eine Verbindung über das Netzwerk 67 zu dem Server-Computer 60 her und lädt eine Eingabemaske für die Anamnese (vgl. Schritt 10 der Figur 1) von dem Anamnese Modul 61. Nach der Speicherung der Anamnesedaten wählt das Programm 66 aus den typischen Betreuungsablauf-Profilen der Datenbank 64 ein passendes Betreuungsablauf-Profil mit entsprechenden Standardeintragungen aus. Dies kann mit Hilfe von in Regeln und/oder Algorithmen gespeichertem medizinischen Expertenwissen erfolgen, d.h. die Anamnese Daten werden von dem Programm 66 ausgewertet, um ein typisches Betreuungsablauf-Profil auszuwählen.

Dieses wird in der Form einer entsprechenden Eingabemaske mittels des Browser-Programms 69 des Client-Computers 68 angezeigt (vgl. Schritt 14 der Figur 1). Nachdem der Patient das typische Betreuungsablauf-Profil modifiziert und / oder ergänzt hat, werden die betreffenden Daten als individuelles Betreuungsablaufprofil, d.h. als "Voreinstellung", in der Datenbank 65 abgespeichert.

Im Weiteren kann der Patient dann über seinen Client-Computer 68 eine Eingabemaske für sein individuelles Betreuungsablauf-Profil abrufen, um dort tatsächliche Betreuungsablauf-Profildaten einzugeben. Die tatsächlichen Betreuungsablauf-Profildaten werden ebenfalls in der Datenbank 65 gespeichert. Diese können mittels des Analysemoduls 62 z.B. entsprechend des Regelsystems 63 untersucht werden. Falls sich bei dieser Analyse eine Risikosituation ergibt, wird der Client-Computer 73 automatisch durch Versenden einer elektronischen Nachricht, beispielsweise einer E-Mail, eines Fax, eine Telefonanrufs oder einer SMS benachrichtigt. Bei dem Client-Computer 73 kann es sich beispielsweise auch um ein Mobiltelefon handeln.

Alternativ kann der Patient auch das tragbare Eingabegerät 71 für die Datenerfassung benutzen. Hierzu wird das tragbare Eingabegerät 71 über die Docking Station 72 mit dem Client-Computer 68 verbunden, um die Eingabemaske für das individuelle Betreuungsablauf-Profil zu laden. Diese kann mittels des Browser-Programms 74 des tragbaren Eingabegeräts 71 angezeigt werden, wie das auch für den Client-Computer 68 der Fall ist.

Das tragbare Eingabegerät 71 wird dann von der Docking Station 72 getrennt und der Patient kann es beispielsweise für eine Reise mit sich führen. Der Benutzer gibt dann seine krankheitsspezifischen Daten in die Eingabemaske seines individuellen Betreuungsablauf-Profils ein, genau so, wie er es auch von seinem Client-Computer 68 gewohnt ist. Im Unterschied zu der Eingabe unmittelbar in den Client-Computer 68 werden jedoch die so eingegebenen Daten zunächst nur lokal in den tragbaren Eingabegerät 71 und zwar in dessen Speicher 75 abgelegt.

Alternativ oder zusätzlich hat das tragbare Eingabegerät 71 eine drahtlose Kommunikationsschnittstelle, beispielsweise für ein Mobiltelefonnetzwerk. Über diese Schnittstelle kann eine Verbindung zwischen dem tragbaren Eingabegerät 71 und dem Server- Computer 60 hergestellt werden, so dass die Synchronisation unmittelbar zwischen dem tragbaren Eingabegerät 71 und dem Server-Computer 60 abläuft. In diesem Fall übernimmt das tragbare Eingabegerät 71 die Funktion den Client-Computers 68. Die Synchronisation kann in diesem Fall aufgrund einer Eingabe des Benutzers, d. h. aufgrund der Betätigung des Buttons 78 erfolgen, oder auch automatisch in fest vorgegebenen Zeitabständen oder zu voreingestellten Zeitpunkten oder dann, wenn eine Einwahl in das Netzwerk durch das tragbare Eingabegerät 71 möglich ist.

Ferner hat das tragbare Eingabegerät 71 einen Sensor 76, der zur Messung des charakteristischen physiologischen Werts dient, also zur Ermittlung des Blutzuckerspiegels im Fall von Diabetes oder zur Messung des sogenannten Peak-Flow im Fall einer Asthma-Erkrankung. Hierbei ist besonders vorteilhaft, dass dieser Messwert vom Benutzer nicht manuell eingegeben werden muss, da er ja unmittelbar in dem tragbaren Eingabegerät 71 vorliegt. Dieser Messwert wird von dem Programm 77 des tragbaren Eingabegerätes 71 zusammen mit den weiteren tatsächlichen Betreuungsablauf-Profildaten in dem Speicher 75 abgelegt.

Nach Rückkehr von seiner Reise stellt der Patient sein tragbaren Eingabegerät 71 in die Docking Station 72 und betätigt den Button 78. Bei dem Button 78 handelt es sich um ein Betätigungselement der Docking Station 72, durch dessen Betätigung das Conduit Programm 70 des Client-Computers 68 gestartet wird. Das Conduit Programm 70 liest automatisch den Inhalt des Speichers 75 aus, startet den Browser 69 und überträgt so die tatsächlichen Betreuungsablauf-Profildaten des tragbaren Eingabegeräts 71 zu dem Server-Computer 60, wo sie in der Datenbank 65 abgespeichert werden. Ebenso kann das Conduit Programm 70 dazu verwendet werden, um Veränderungen, die der Benutzer zwischenzeitlich hinsichtlich seines individuellen Betreuungsablauf-Profils vorgenommen hat, mit dem Server-Computer 60 zu synchronisieren, und die entsprechenden Veränderungen in der Datenbank 65 abzuspeichern.

Das Computersystem der Figur 16 ist also für den Benutzer in mehrfacher Hinsicht besonders vorteilhaft. Zum einen erlaubt es dem Benutzer eine bequeme Registrierung und ferner eine effiziente fortlaufende Datenerfassung, die zudem maximale Mobilität erlaubt.

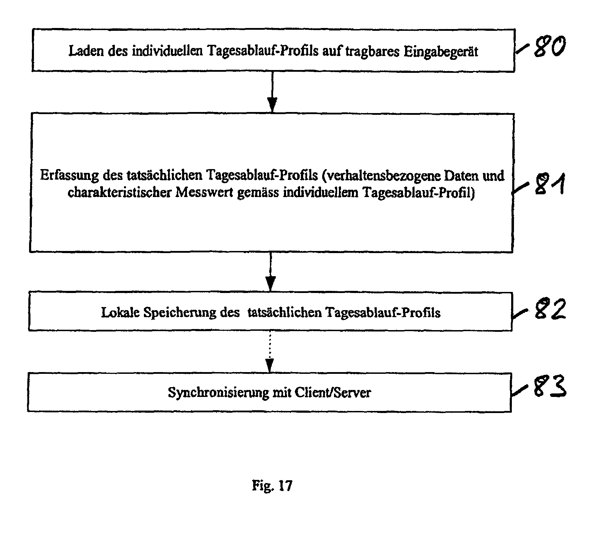

Die Figur 17 zeigt ein entsprechendes Flussdiagramm für den Betrieb des tragbaren Eingabegeräts. In dem Schritt 80 wird zunächst das zuvor von dem Benutzer gespeicherte individuelle Betreuungsablauf-Profil auf das tragbare Eingabegerät übertragen, so dass eine entsprechende Eingabemaske auf dem tragbaren Eingabegerät zur Verfügung steht.

In dem Schritt 81 erfasst der Patient die tatsächlichen Betreuungsablauf-Profildaten mittels dieser Eingabemaske, das heißt, insbesondere seiner verhaltensbezogenen Daten. Die charakteristischen Messwerte werden ebenfalls mit dem Eingabegerät gemessen. Der oder die Messwerte werden zusammen mit den verhaltensbezogenen Daten jeweils als tatsächliche Betreuungsablauf-Profildaten abgespeichert.

Diese Abspeicherung erfolgt in dem Schritt 82 lokal in dem tragbaren Eingabegerät. Dieser Vorgang der Schritte 81 und 82 kann sich täglich mehrfach wiederholen, um so zu einem vollständigen tatsächlichen Betreuungsablauf-Profil zu kommen oder er kann auch -je nach der Art der chronischen Erkrankung - in größeren Zeitabständen vorgenommen werden.

In dem Schritt 83 erfolgt dann die Synchronisierung mit dem Client-Computer und / oder dem Server-Computer zur Übertragung der zunächst nur lokal gespeicherten tatsächlichen Betreuungsablauf-Profildaten.

### Bezugszeichenliste

- Eingabemaske: 42
- Eingabemaske: 43
- Eingabemaske: 44
- Bedienelement: 45
- Bedienelement: 46
- Eingabemaske: 47
- Bedienelement: 48
- Eingabemaske: 49
- Eingabemaske: 50
- Eingabemaske: 51
- Eingabemaske: 52
- Kalender-Feld: 53
- Eingabemaske: 54
- Eingabemaske: 55
- Grafik: 56
- Normalbereich: 57
- Grenzbereich: 58
- Risikobereich: 59
- Server-Computer: 60
- Anamnese Modul: 61
- Analyse Modul: 62
- Regelsystem: 63
- Datenbank: 64
- Datenbank: 65
- Programm: 66
- Netzwerk: 67
- Client-Computer: 68
- Browser Programm: 69
- Conduit: 70
- tragbares Eingabegerät: 71
- Docking Station: 72
- Client-Computer: 73
- Browser Programm: 74
- Speicher: 75
- Sensor: 76
- Programm: 77
- Button: 78

## Patentansprüche

1. Verfahren zur Datenerfassung für die Ermittlung des Verlaufs einer chronischen Erkrankung mit folgenden Schritten:
- Eingabe (11) von Anamnesedaten,
- Auswahl (13) eines typischen Betreuungsablauf-Profils, welches den Anamnesedaten entspricht, unter Verwendung formalisierten medizinischen Fachwissens, welches von einer Verarbeitungsmaschine angewendet wird,
- Eingabe (17) von Daten zur Modifikation und / oder Ergänzung des typischen Ablauf-Profils zur Erzeugung eines individuellen Ablauf-Profils,
- Anzeige (21) einer Eingabemaske mit dem individuellen Ablauf-Profil mit Standardeintragungen,
- Eingabe (23) von zumindest einem aktuellen charakteristischen Messwert hinsichtlich der chronischen Erkrankung,
- bei einer Abweichung des tatsächlichen Ablauf-Profils von dem individuellen Ablauf-Profil: Eingabe (24) von Daten zur Modifikation und / oder Ergänzung des individuellen Ablauf-Profils zur Abgleichung mit dem tatsächlichen Ablauf-Profil,
- Speicherung (25) der tatsächlichen Ablauf-Profildaten in einer Datenbank.

2. Verfahren nach Anspruch 1, wobei die Anamnesedaten Daten zur Angabe eines verordneten Therapieverfahrens beinhalten und die automatische Auswahl eines typischen Ablauf-Profils mittels der Daten zur Angabe eines verordneten Therapieverfahrens erfolgt.

3. Verfahren nach Anspruch 1 oder 2 mit folgenden weiteren Schritten:
- Anzeige (14) einer Eingabemaske mit dem automatisch ausgewählten typischen Ablauf-Profil mit Eingabefeldern, die mit Standardeinträgen vorbelegt sind, wobei es sich bei den Standardeinträgen insbesondere um Zeitpunkte handelt, zu denen ein bestimmtes Verhalten des Patienten typischerweise erfolgt,
- Editierung (17) der Standardeinträge zur Erzeugung eines individuellen Ablauf-Profils, welches von dem typischen Ablauf-Profil abweichend ist,
- Speicherung (18) des individuellen Ablauf-Profils mit den editierten Standardeintragungen.

4. Verfahren nach Anspruch 1, 2 oder 3, wobei es sich bei dem Verhalten des Patienten um die Einnahme einer bestimmten Mahlzeit, die Einnahme von Medikamenten, körperliche Bewegung oder ein anderes hinsichtlich der chronischen Erkrankung relevantes Verhalten zu einem bestimmten Zeitpunkt handelt.

5. Verfahren nach einem der vorhergehenden Ansprüche 1 bis 4 mit folgenden weiteren Schritten:
- Laden (80) des individuellen Ablauf-Profils auf ein tragbares Eingabegerät von einem Computer,
- Eingabe (81) des tatsächlichen Ablauf-Profils in das Eingabegerät,
- Messung des aktuellen charakteristischen Messwerts mit dem Eingabegerät,
- lokale Speicherung (82) der tatsächlichen Ablauf-Profildaten in dem Eingabegerät,
- Synchronisation (83) der in dem Eingabegerät lokal gespeicherten tatsächlichen Ablauf-Profildaten mit dem Computer.

6. Verfahren nach einem der vorhergehenden Ansprüche 1 bis 5, wobei es sich bei dem Computer um einen Server-Computer oder um einen Host-Computer handelt.

7. Verfahren nach einem der vorhergehenden Ansprüche 1 bis 6 mit folgenden weiteren Schritten:
- Prüfung (32), ob die tatsächlichen Ablauf-Profildaten eine erste Regel oder einen ersten Regelsatz erfüllen,
- Prüfung (34), ob die tatsächlichen Ablauf-Profildaten eine zweite Regel oder einen zweiten Regelsatz erfüllen,
- Prüfung (38), ob die tatsächlichen Ablauf-Profildaten, welche die zweite Regel oder den zweiten Regelsatz erfüllen, auch eine vierte Regel oder einen vierten Regelsatz erfüllen,
- Markierung (33, 35, 39) der tatsächlichen Ablauf-Profildaten mit einem ersten, zweiten oder dritten Symbol, wenn die betreffenden tatsächlichen Ablauf-Profildaten die erste, zweite oder zweite und vierte Regel bzw. die entsprechenden Regelsätze erfüllen.

8. Computersystem zur Datenerfassung für die Ermittlung des Verlaufs einer chronischen Erkrankung mit
- ersten Datenbankmitteln (61) zur Eingabe von Anamnesedaten,
- zweiten Datenbankmitteln (64) zur Speicherung von typischen Ablauf-Profilen für verschiedene chronische Erkrankungen und / oder verschiedene Therapieverfahren für eine chronische Erkrankung,
- Programmmitteln (66) zur Auswahl eines typischen Ablauf-Profils aus den zweiten Datenbankmitteln mittels der Anamnesedaten,
- Mitteln (69) zur Anzeige einer Eingabemaske mit einem typischen Ablauf-Profil für einen Patienten, wobei das typische Ablauf-Profil Standardeintragungen hinsichtlich eines typischen Verhaltens des Patienten beinhaltet,
- Mitteln (69) zur Editierung der Standardeintragung durch den Patienten und zur Eingabe von Daten zur Modifikation und / oder Ergänzung des typischen Ablauf-Profils zur Erzeugung eines individuellen Ablauf-Profils,
- dritten Datenbankmitteln (65) zur Speicherung des individuellen Ablauf-Profils.

9. Computersystem nach Anspruch 8 mit
- Mitteln (69) zur Anzeige einer Eingabemaske mit dem individuellen Ablauf-Profil, wobei das individuelle Ablauf-Profil die gegebenenfalls editierten Standardeintragungen beinhaltet,
- Mitteln (69) zur Eingabe des tatsächlichen Ablauf-Profils in die Eingabemaske zur Abgleichung des individuellen Ablauf-Profils mit dem tatsächlichen Verhalten des Patienten und zur Eingabe von zumindest einem aktuellen charakteristischen Messwert.

10. Tragbares Eingabegerät für ein Computersystem nach Anspruch 8 oder 9 mit
- Mitteln (77) zum Laden des individuellen Ablauf-Profils von dem Computersystem,
- Mitteln (74) zur Eingabe des tatsächlichen Ablauf-Profils,
- Mitteln (76) zur Messung des aktuellen charakteristischen Messwerts,
- Mitteln (75) zur lokalen Speicherung der tatsächlichen Ablauf-Profildaten und des charakteristischen Messwerts,
- Mitteln (72) zur Synchronisation mit dem Computersystem, wobei die Mittel zur Synchronisation vorzugsweise ein einzelnes Bedienelement zur Einleitung der Synchronisation aufweisen.

11. Computerprogramm zur Durchführung eines Verfahrens nach einem der vorhergehenden Ansprüche 1 bis 7.

## Claims

1. Method of data acquisition for determining the progress of a chronic disease, with the following stages:
- input (11) of anamnesis data,
- selection (13) of a typical care process profile corresponding to the anamnesis data, using formalised medical expert knowledge which is used by a processing machine,
- input (17) of data on modifying and/or supplementing the typical process profile for generating an individual process profile,
- display (21) of an input mask with the individual process profile, with standard entries,
- input (23) of at least one current characteristic measured value relating to the chronic disease,
- in the event of a deviation of the actual process profile from the individual process profile: input (24) of data on the modification and/or supplementing of the individual process profile for harmonisation with the actual process profile,
- storage (25) of the actual process profile data in a database.

2. Method according to Claim 1, wherein the anamnesis data include data for indicating a prescribed therapy method and the automatic selection of a typical process profile is made by means of the data for indicating a prescribed therapy method.

3. Method according to Claim 1 or 2 with the following further stages:
- display (14) of an input mask with the automatically selected typical process profile with input fields which are filled with standard entries, whereupon the standard entries include, in particular, times when a certain behaviour of the patient typically takes place,
- editing (17) of the standard entries to generate an individual process profile with the edited standard entries.

4. Method according to Claim 1, 2 or 3, wherein the behaviour of the patient includes the taking of a certain meal, the taking of medicines, physical movement or any other behaviour relevant to the chronic disease at a certain time.

5. Method according to one of the preceding Claims 1 to 4, with the following further stages:
- loading (80) of the individual process profile into a portable input device of a computer,
- input (81) of the actual process profile into the input device,
- measurement of the current characteristic measured value with the input device,
- local storage (82) of the actual process profile data in the input device,
- synchronisation (83) of the actual process profile data stored locally in the input device with the computer.

6. Method according to one of the preceding Claims 1 to 5, wherein the computer is a server computer or a host computer.

7. Method according to one of the preceding Claims 1 to 6, with the following further stages:
- checking (32) to determine whether the actual process profile data conform to a first rule or a first set of rules,
- checking (34) to determine whether the actual process profile data conform to a second rule or a second set of rules,
- checking (38) to determine whether the actual process profile data which conform to the second rule or the second set of rules and also conform to a fourth rule or a fourth set of rules,
- marking (33, 35, 39) of the actual process profile data with a first, second or third symbol when the corresponding actual process profile data conform to the first, second or second and fourth rules and the corresponding sets of rules.

8. Computer system for data acquisition for determining the progress of a chronic disease with
- first database means (61) for inputting anamnesis data,
- second database means (64) for storing typical process profiles for different chronic diseases and/or different therapy methods for a chronic disease,
- program means (66) for selecting a typical process profile from the second database means on the basis of the anamnesis data,
- means (69) for displaying an input mask with a typical process profile, wherein the typical process profile incorporates standard entries regarding a typical behaviour of the patient,
- means (69) for editing the standard entry by the patient and for inputting data on the modification and/or supplementing of the typical process profile to generate an individual process profile,
- third database means (65) for storing the individual process profile.

9. Computer system according to Claim 8, with
- means (69) for displaying an input mask with the individual process profile, wherein the individual process profile incorporates any edited standard entries,
- means (69) for inputting the actual process file into the input mask to harmonise the individual process profile with the actual behaviour of the patient and for inputting at least one actual characteristic measured value.

10. Portable input device for a computer system according to Claim 8 or 9, with
- means (77) for loading the individual process profile from the computer system,
- means (74) for inputting the actual process profile,
- means (76) for measuring the current characteristic measured value,
- means (75) for local storage of the actual process profile data and of the characteristic measured value,
- means (72) for synchronising with the computer system, wherein the means for synchronisation preferably exhibit an individual operating element for introducing the synchronisation.

11. Computer program for carrying out a method according to one of the preceding Claims 1 to 7.

## Revendications

1. Procédé de saisie de données pour déterminer l'évolution d'une maladie chronique avec les étapes suivantes :
- saisie (11) des données d'anamnèse,
- sélection (13) d'un profil typique du processus de l'assistance médicale, lequel profil correspond aux données d'anamnèse, sous réserve d'utilisation des connaissances médicales formalisées appliquées par une machine de traitement,
- introduction (17) de données pour modifier et/ou compléter le profil de processus typique permettant de générer un profil de processus individuel,
- affichage (21) d'un masque de saisie avec le profil de processus individuel comportant les entrées standards,
- introduction (23) d'au moins une valeur mesurée caractéristique et actuelle par rapport à la maladie chronique,
- en cas d'écart du profil de processus effectif par rapport au profil de processus individuel : introduction (24) de données pour modifier et/ou compléter le profil de processus individuel afin de compenser avec le profil de processus effectif,
- enregistrement (25) des données du profil de processus effectif dans une base de données.

2. Procédé selon la revendication 1,
dans lequel
les données d'anamnèse comprennent des données relatives à un traitement thérapeutique prescrit et la sélection automatique d'un profil de processus typique a lieu à l'aide des données relatives à un traitement thérapeutique prescrit.

3. Procédé selon la revendication 1 ou 2 avec les étapes suivantes :
- affichage (14) d'un masque de saisie avec le profil de processus typique sélectionné automatiquement comportant les champs d'introduction des données pré-allouées avec des saisies standards, les saisies standards étant principalement des moments au cours desquels un comportement spécifique du patient a typiquement lieu,
- édition (17) des saisies standards pour la création d'un profil de processus individuel qui diverge du profil de processus typique,
- enregistrement (18) du profil de processus individuel avec les saisies standards éditées.

4. Procédé selon la revendication 1, 2 ou 3,
dans lequel
le comportement du patient correspond à la prise d'un certain repas ou de médicaments, au déplacement physique ou à tout autre comportement pertinent par rapport à la maladie chronique à un moment donné.

5. Procédé selon l'une des revendications précédentes 1 à 4 avec les étapes suivantes :
- chargement (80) du profil de processus individuel sur une unité d'entré portative d'un ordinateur,
- introduction (81) du profil de processus effectif dans l'appareil d'entrée,
- mesure de la valeur mesurée caractéristique avec l'appareil d'entrée,
- enregistrement local (82) des données du profil de processus effectif dans un appareil d'entrée,
- synchronisation (83) des données du profil de processus effectif enregistré localement dans l'appareil d'entrée avec l'ordinateur.

6. Procédé selon l'une des revendications précédentes 1 à 5,
dans lequel
l'ordinateur est un ordinateur serveur ou hôte.

7. Procédé selon l'une des revendications précédentes 1 à 6 avec les étapes suivantes :
- vérification (32) visant à déterminer si les données du profil de processus effectif remplissent une première règle ou un premier groupe de règles,
- vérification (34) visant à déterminer si les données du profil de processus effectif remplissent une deuxième règle ou un deuxième groupe de règles,
- vérification (38) visant à déterminer si les données du profil de processus effectif qui remplissent la deuxième règle ou le deuxième groupe de règles, remplissent également une quatrième règle ou un quatrième groupe de règle,
- repérage (33, 35, 39) des données du profil de processus effectif avec un premier, un deuxième ou un troisième symbole lorsque les données du profil de processus effectif concernées remplissent la première, deuxième ou deuxième et quatrième règle ou le groupe de règles correspondant.

8. Système informatique de saisie des données pour déterminer l'évolution d'une maladie chronique avec
- des premiers moyens de base de données (61) pour saisir les données d'anamnèse,
- des deuxièmes moyens de base de données (64) pour enregistrer les profils de processus typiques pour différentes maladies chroniques et/ou différents traitements thérapeutiques pour une maladie chronique,
- des moyens de programmation (66) pour sélectionner un profil de processus typique à partir des deuxièmes moyens de base de données à l'aide des données d'anamnèse,
- des moyens (69) pour afficher un masque de saisie avec un profil de processus typique pour un patient, le profil de processus typique comportant des saisies standards relatives à un comportement typique du patient,
- des moyens (69) pour éditer la saisie standard par le patient et pour entrer les données visant à modifier et/ou à compléter le profil de processus typique pour générer un profil de processus individuel,
- des troisièmes moyens de base de données (65) pour enregistrer le profil de processus individuel.

9. Système informatique selon la revendication 8,
avec
- des moyens (69) pour afficher un masque de saisie avec le profil de processus individuel, le profil de processus individuel comprenant les saisies standards éditées le cas échéant,
- des moyens (69) pour introduire le profil de processus effectif dans le masque de saisie afin de compenser le profil de processus individuel avec le comportement effectif du patient et d'entrer au moins une valeur mesurée actuelle caractéristique.

10. Appareil d'entrée portatif pour un système informatique selon la revendication 8 ou 9 avec
- des moyens (77) pour charger le profil de processus individuel du système informatique,
- des moyens (74) pour introduire le profil de processus effectif,
- des moyens (76) pour mesurer la valeur mesurée actuelle caractéristique,
- des moyens (75) pour enregistrer localement les données du profil de processus effectif et de la valeur mesurée caractéristique,
- des moyens (72) pour se synchroniser avec le système informatique, les moyens de synchronisation présentant de préférence un seul organe de commande pour introduire la synchronisation.

11. Programme informatique pour effectuer un procédé selon l'une des revendications précédentes 1 à 7.
